# FASCICULE DE BREVET EUROPEEN

(11) **EP 0 832 081 B1**
(45) Date de publication et mention de la délivrance du brevet: **29.01.2003**
(21) Numéro de dépôt: 97908308.6
(22) Date de dépôt: 05.03.1997
(51) Int. Cl.: C07D 333/24, C07D 333/16, C07D 307/54, C07D 207/32, C07D 213/55, C07C 69/618, A61K 31/38, A61K 31/19

(54) **COMPOS S BICYCLIQUES-AROMATIQUES**
BICYCLISCHE AROMATISCHE VERBINDUNGEN
BICYCLIC-AROMATIC COMPOUNDS

(30) Priorité: 14.03.1996 FR 9603235
(43) Date de publication de la demande: 01.04.1998
(73) Titulaire: CENTRE INTERNATIONAL DE RECHERCHES DERMATOLOGIQUES GALDERMA, ( CIRD GALDERMA), 06560 Valbonne (FR)
(72) Inventeur: BERNARDON, Jean-Michel, F-06650 Le Rouret (FR)
(74) Mandataire: Tezier Herman, Béatrice
(86) Numéro de dépôt international: FR9700391
(87) Numéro de publication internationale: WO97033881

(56) Documents cités:
- EP-A- 0 260 162
- EP-A- 0 679 630
- EP-A- 0 722 928
- US-A- 4 792 567
- CHEMICAL ABSTRACTS, vol. 80, no. 7, 18 Février 1974 Columbus, Ohio, US; abstract no. 36614, J. KOVAC ET AL.: "Thiophene derivatives. Alpha, Beta-Unsaturated ketones of phenylthiophene series." XP002020611 & CHEM. ZVESTI, vol. 27, no. 4, 1973, CZECH., pages 512-520,
- CHEMICAL ABSTRACTS, vol. 80, no. 19, 13 Mai 1974 Columbus, Ohio, US; abstract no. 108280, V.K. POLYAKOV ET AL.: "Carbonyl derivatives of 2-arylthiophenes." XP002020612 & KHIM. GETEROTSIKL. SOEDIN., no. 1, 1974, USSR, pages 136-137,
- CHEMISCHE BERICHTE, vol. 108, no. 4, 1975, DE, pages 1040-1042, XP002020610

## Description

L'invention concerne, à titre de produits industriels nouveaux et utiles, des composés bicycliques-aromatiques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Différents documents de l'état de la technique proposaient des composés bicycliques aromatiques tels que Kovac *et al*. dans « Thiophene derivatives. Alpha, Beta-Unsaturaetd ketones of phenylthiophene series » voir Chemical Abstracts, vol. 80, N°7 du 18 février 1974, (abs. 36614), EP-A-0 679 630, EP-A-0 260 162, US 4,792,567, EP-A-0 722 928, Polyakov et al. « Carbonyl derivatives of 2-arylthiophernes » voir Chemical Abstract, vol. 8, N°19 du 13 mai 1974 (abs. 108280) et Chemische Berichte, vol. 108, N°4, 1975, p. 1040-1042.
Ces composés prénsentent néanmoins des structures différentes, certains sont proposés pour des applications pharmaceutiques ou cosmétiques sans pour autant présenter des activités d'agonistes des récepteurs RXR comparables aux composés de la présente invention.

Les composés selon l'invention peuvent être représentés par la formule générale (I) suivante : 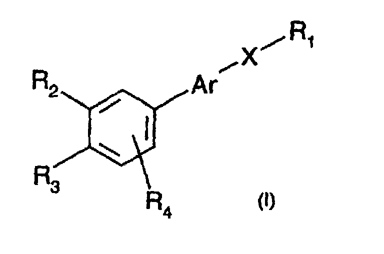 dans laquelle:
- R₁ représente
   (i) le radical -CH₃
   (ii) le radical -CH₂OR₅
   (iii) le radical -CO-R₆
   R₅ et R₆ ayant la signification donnée ci-après
- Ar représente un radical choisi parmi les radicaux de formules (a)-(e) suivantes: 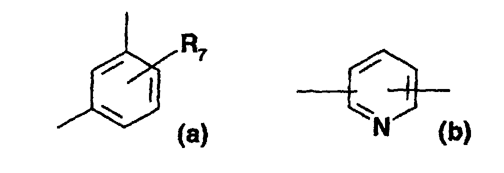 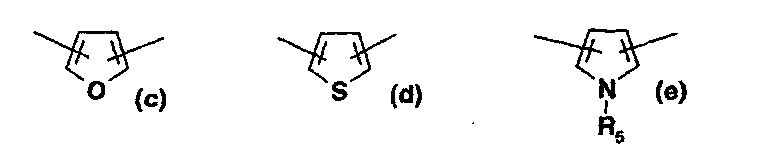 R₅ et R₇ ayant la signification donnée ci-après,
- X représente 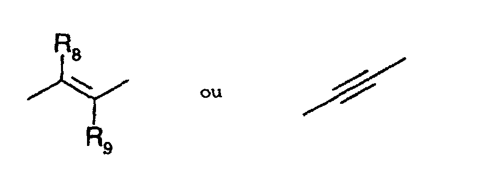 R₈ et R₉ ayant les significations données ci-après
- R₂ et R₃, identiques ou différents, représentent
   (i) un atome d'hydrogène,
   (ii) un radical alkyle présentant au moins 3 atomes de carbone, parmi lesquels le carbone attaché au radical phényl est au moins substitué par deux atomes de carbone,
   (iii) un radical -OR₅,
   (iv) un radical -SR₅,
   R₅ ayant la signification donnée ci-après,
   étant entendu que R₂ et R₃ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
   et étant entendu que R₂ et R₃ ne peuvent pas avoir en même temps les significations (i), (iii) et (iv) mentionnées ci-dessus.
- R₄ et R₇, identiques ou différents, représentent un atome d'hydrogène, un atome d'halogène, un radical alkyle linéaire ou ramifié ayant de 1 à 20 atomes de carbone, un radical -OR₅,
- R₅ représente un atome d'hydrogène, un radical alkyle ayant de 1 à 12, de préférence de 1 à 9, atomes de carbone ou un radical -COR₁₀
   R₁₀ ayant la signification donnée ci-après,
- R₆ représente:
   (a) un atome d'hydrogène
   (b) un radical alkyle ayant de 1 à 12, de préférence de 1 à 9, atomes de carbone
   (c) un radical de formule: 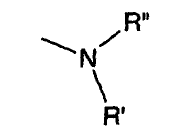 R' et R" ayant la signification donnée ci-après,
   (d) un radical -OR₁₁
   R₁₁ ayant la signification donnée ci-après,
- R₈ et R₉, identiques ou différents, représentent un atome d'hydrogène ou un radical alkyle ayant de 1 à 12, de préférence de 1 à 9, atomes de carbone,
- R₁₀ représente un radical alkyle ayant de 1 à 12, de préférence de 1 à 9, atomes de carbone,
- R₁₁ représente un atome d'hydrogène, un radical alkyle linéaire ou ramifié, ayant de 1 à 20 atomes de carbone, un radical alkényl, un radical mono ou polyhydroxyalkyle, un radical aryle ou aralkyle, éventuellement substitué(s) par au moins un atome d'halogène, un hydroxyle ou une fonction nitro ou un reste de sucre ou un reste d'amino acide ou de peptide,
- R' et R", identiques ou différents, représentent un atome d'hydrogène, un radical alkyl ayant de 1 à 12, de préférence de 1 à 9, atomes de carbone, un radical mono ou polyhydroxyalkyle, un radical aryle éventuellement substitué par au moins un atome d'halogène, un hydroxyle ou une fonction nitro ou un reste d'amino acide ou de sucre ou encore pris ensemble forment un hétérocycle.

L'invention vise également les sels des composés de formule (I) lorsque R₁ représente une fonction acide carboxylique et les isomères géométriques et optiques desdits composés de formule (I).

Lorsque les composés selon l'invention se présentent sous forme de sels, il s'agit de préférence de sels d'un métal alcalin ou alcalino-terreux, ou encore de zinc ou d'une aminé organique.

Par radical alkyle linéaire ayant de 1 à 20 atomes de carbone, on entend notamment les radicaux méthyle, éthyle, propyle, pentyle, hexyle, octyle, décyle, dodécyle, hexadécyle et octadécyl.

Parmi les radicaux alkyles ayant de 1 à 12, de préférence de 1 à 9, atomes de carbone, on préfère les radicaux méthyle, éthyle, propyle, isopropyle, butyle, tertiobutyle, pentyle, hexyle, heptyle, nonyle, décyle et dodécyle.

Par radical alkyle ramifié ayant de 1 à 20 atomes de carbone, on entend notamment les radicaux 2-éthylhexyle, 2-méthylbutyle, 2-méthylpentyle, 1-méthylhexyle, 3-méthylheptyle.

Parmi les radicaux alkyle présentant au moins 3 atomes de carbone, où le carbone attaché au radical phényl est au moins substitué par deux atomes de carbone, on peut citer le radical tertiobutyle, isopropyle, 1,1-diméthylhexyle et 1,1-diméthyldécyle. De préférence, ces radicaux présentent au maximum 20 atomes de carbone, encore plus préférentiellement au maximum 12 atomes de carbone. De manière avantageuse, le radical (ii) est le radical tertiobutyle.

Parmi les radicaux monohydroxyalkyle, on préfère un radical présentant 2 ou 3 atomes de carbone, notamment un radical 2-hydroxyéthyle, 2-hydroxypropyle ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle substitué, on préfère un radical phényle substitué par au moins un atome d'halogène, un hydroxyle ou une fonction nitro.

Parmi les radicaux aralkyle substitué, on préfère le radical benzyle ou phénéthyle substitué par au moins un atome d'halogène, un hydroxyle ou une fonction nitro.

Parmi les radicaux alkényle, on préfère un radical contenant de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque les radicaux R₄ et R₇ représentent un atome d'halogène, celui-ci est de préférence un atome de fluor, de brome ou de chlore.

Parmi les composés de formule (I) ci-dessus rentrant dans le cadre de la présente invention, on peut notamment citer les suivants :
- Acide 5-(3-tert-butyl-4-méthoxyphényl)-2-thiophèneacrylique,
- Acide 5-(3-tert-butyl-4-méthoxyphényl)-2-thiophènepropiolique,
- Acide 2-(3-tert-butyl-4-méthoxyphényl)-4-thiophèneacrylique,
- Acide 4-(3-tert-butyl-4-méthoxyphényl)-2-thiophèneacrylique,
- Acide 5-(3,5,5,8,8-pentaméthyl-5,6,7,8-tétrahydro-2-naphtyl)-2-thiophène acrylique,
- Acide 4-(3,5,5,8,8-pentaméthyl-5,6,7,8-tétrahydro-2-naphtyl)-2-thiophène acrylique,
- Acide 4-(5,6,7,8-tétrahydro-5,5,8,8-tétraméthyl-2-naphtyl)-2-thiophene acrylique,
- Acide 5-(3,5,5,8,8-pentaméthyl-5,6,7,8-tétrahydro-2-naphtyl)-2-thiophène propiolique,
- Acide 3-(3,5,5,8,8-pentaméthyl-5,6,7,8-tétrahydro-2-naphtyl)phenyl propiolique,
- Acide N-méthyl-4-(5,6,7,8-tétrahydro-5,5,8,8-tétraméthyl-2-naphtyl)-2-pyrroleacrylique,
- Acide 3-(5,6,7,8-tétrahydro-5,5,8,8-tétraméthyl-2-naphtyl)phenylacrylique,
- Acide N-méthyl-4-(3,5,5,8,8-pentaméthyl-5,6,7,8-tétrahydro-2-naphtyl)-2-pyrroleacrylique,
- Acide 4-(3,5,5,8,8-pentaméthyl-5,6,7,8-tétrahydro-2-naphtyl)-2-pyrrole acrylique,
- Acide 3-(3,5,5,8,8-pentaméthyl-5,6,7,8-tétrahydro-2-naphtyl)phenylacrylique,
- Acide 3-(5,6,7,8-tétrahydro-5,5,8,8-tétraméthyl2-naphtyl)phenylpropiolique,
- Acide 2-methoxy-3-(3,5,5,8,8-pentaméthyl-5,6,7,8-tétrahydro-2-naphtyl) phenylacrylique,
- Acide 2-propyloxy-3-(3,5,5,8,8-pentaméthyl-5,6,7,8-tétrahydro-2-naphtyl) phenylacrylique,
- Acide2-heptyloxy-3-(3,5,5,8,8-pentaméthyl-5,6,7,8-tétrahydro-2-naphtyl) phenylacrylique,
- Acide 2-methoxymethoxy-3-(3,5,5,8,8-pentaméthyl-5,6,7,8-tétrahydro-2-naphtyl) phenylacrylique,
- Acide 2-hydroy-3-(3,5,5,8,8-pentaméthyl-5,6,7,8-tétrahydro-2-naphtyl) phenylacrylique,
- Acide 3-(3-methoxy-5,6,7,8-tétrahydro-5,5,8,8-tétraméthyl-2-naphtyl) phenylacrylique,
- Acide 3-(3-propyloxy-5,6,7,8-tétrahydro-5,5,8,8-tétraméthyl-2-naphtyl) phenylacrylique,
- Acide 3-(3-heptyloxy-5,6,7,8-tétrahydro-5,5,8,8-tétraméthyl-2-naphtyl) phenylacrylique,
- Acide 3-(3-methoxymethoxy-5,6,7,8-tétrahydro-5,5,8,8-tétraméthyl-2-naphtyl)phenylacrylique,
- Acide 3-(3-hydroxy-5,6,7,8-tétrahydro-5,5,8,8-tétraméthyl-2-naphtyl) phenylacrylique,
- Acide 3-(4,4,7-trimethylthiochromane-6-yl)phenylacrylique,
- N-ethyl-3-(3,5,5,8,8-pentaméthyl-5,6,7,8-tétrahydro-2-naphtyl)phenyl acrylamide,
- N-(4-hydroxyphenyl)-3-(3,5,5,8,8-pentaméthyl-5,6,7,8-tétrahydro-2-naphtyl) phenylacrylamide,
- Morpholide de l'acide 3-(3,5,5,8,8-pentaméthyl-5,6,7,8-tétrahydro-2-naphtyl)phenylacrylique,
- 3-(3,5,5,8,8-pentaméthyl-5,6,7,8-tétrahydro-2-naphtyl)phenylacrylate d'éthyle,
- Acide 3-[3-(3,5,5,8,8-pentaméthyl-5,6,7,8-tétrahydro-2-naphtyl)phenyl]but-2-enoique,
- Acide 4-methoxymethoxy-3-(3,5,5,8,8-pentaméthyl-5,6,7,8-tétrahydro-2-naphtyl)phenylacrylique,
- Acide 4-hydroxy-3-(3,5,5,8,8-pentaméthyl-5,6,7,8-tétrahydro-2-naphtyl) phenylacrylique,
- Acide 4-methoxy-3-(3,5,5,8,8-pentaméthyl-5,6,7,8-tétrahydro-2-naphtyl) phenylacrylique,
- Acide 4-propyloxy-3-(3,5,5,8,8-pentaméthyl-5,6,7,8-tétrahydro-2-naphtyl) phenylacrylique,
- Acide 4-heptyloxy-3-(3,5,5,8,8-pentaméthyl-5,6,7,8-tétrahydro-2-naphtyl) phenylacrylique,
- 3-(3,5,5,8,8-pentaméthyl-5,6,7,8-tétrahydro-2-naphtyl)phenyl]acroleine,
- 3-[ 3-(3,5,5,8,8-pentaméthyl-5,6,7,8-tétrahydro-2-naphtyl)phenyl]prop-2-en-1-ol,
- Acide *cis*-3-[3-(3,5,5,8,8-pentaméthyl-5,6,7,8-tétrahydro-2-naphtyl)phenyl] but-2-enoique,
- Acide *cis*-3-(3,5,5,8,8-pentaméthyl-5,6,7,8-tétrahydro-2-naphtyl)phenyl acrylique,
- Acide 5-(3,5,5,8,8-pentaméthyl-5,6,7,8-tétrahydro-2-naphtyl)-3-pyridine acrylique,
- Acide -3-(3-butyl-5,6,7,8-tétrahydro-5,5,8,8-tétraméthyl-2-naphtyl)phenyl acrylique,
- Acide 6-(3,5,5,8,8-pentaméthyl-5,6,7,8-tétrahydro-2-naphtyl)-2-pyridine acrylique.

Selon la présente invention les composés de formule (I) plus particulièrement préférés sont ceux pour lesquels au moins l'une des, et de préférence toutes les, conditions suivantes est remplie :
- R₁ représente le radical -CO-R₆
- Ar représente les radicaux de formule (a) ou (d)
- X représente le radical 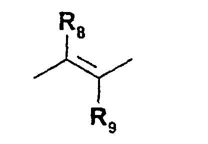
- R₂ et R₃ pris ensemble forment avec le cycle aromatique adjacent un cycle à 5 ou 6 chainons éventuellement substitué par des groupes méthyle et/ou éventuellement interrompu par un atome d'oxygène ou de soufre.

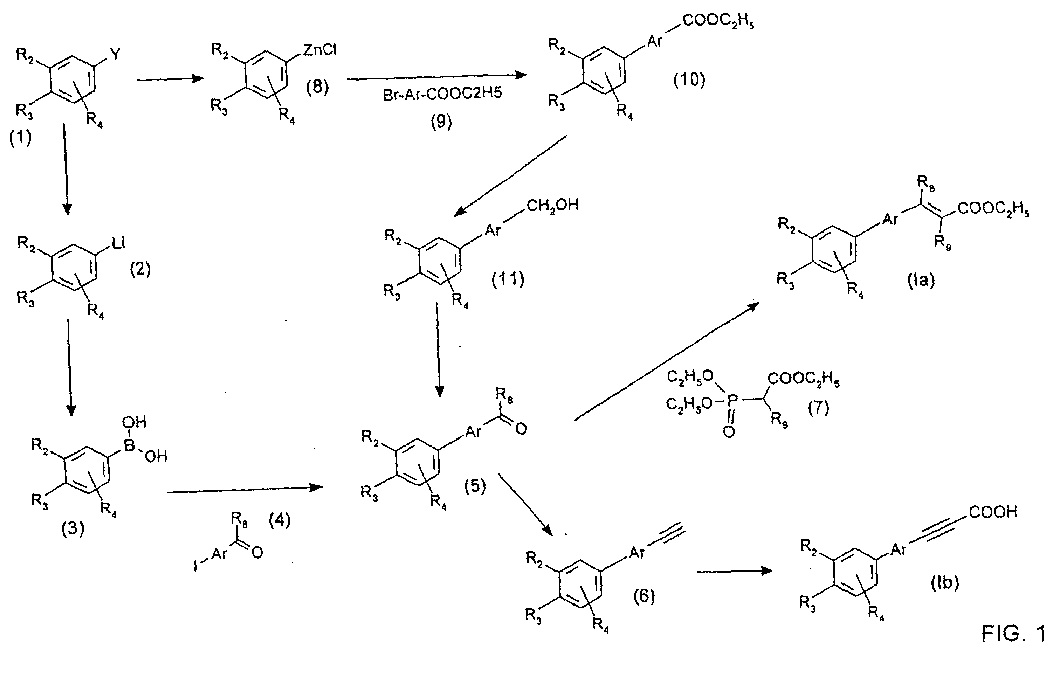

La présente invention a également pour objet les procédés de préparation des composés de formule (I), en particulier selon le schéma réactionnel donné à la figure 1.

Ainsi, les dérivés de formule (la) peuvent être obtenus (FIG. 1) à partir des dérivés aldéhydiques ou cétoniques (5), selon une réaction de type Horner avec un lithio ou sodio dérivé d'un phosphonate (7). Les composés carbonylés (5) pouvant être obtenus:
- soit par une réaction de couplage entre un acide boronique (3) et un dérivé halogéné (4). Cette réaction est effectuée en présence d'un catalyseur au palladium, par exemple le tétrakis(triphénylphosphine)palladium selon les conditions décrites par N. Miyaura et al. Synthetic Communications (1981) 11(7), 513-519.Le dérivé acide boronique (3) pouvant être obtenu par exemple, à partir du dérivé halogéné (1) par transformation en lithien (2) puis réaction avec le triméthyl borate et hydrolyse.
- soit par une réaction de couplage entre un dérivé zincique (8) et un dérivé ester-halogéné (9) en présence d'un catalyseur par exemple un dérivé du palladium ou de nickel (NiCl₂ dppe) puis transformation de la fonction ester (10) en alcool (11) et oxydation en aldéhyde (5).

Les composés de formule (lb) peuvent être obtenus (FIG. 1) à partir du dérivé acétylénique (6) par réaction avec le n-butyl lithium puis carboxylation en présence de CO₂. Les composés acétyléniques (6) pouvant être obtenus soit:
- à partir des dérivés aldéhydiques (5) (lorsque R₈ est un atome d'hydrogène), par réaction avec le tétrabromure de carbone et la triphenylphosphine pour donner un dérivé 2',2'-dibromostyrene qui est transformé en dérivé acétylénique par une base non nucléophile tel le n-butyllihium dans un solvant aprotic tel le tetrahydrofuranne.
- à partir des dérivés cétoniques (5) (lorsque R₈ est un alkyl inférieur) par une suite de réactions comprenant le traitement avec une base tel le diisopropylamidure de lithium puis avec un chlorure de dialkylphosphate et de nouveau avec le diisopropylamidure de lithium.

Lorsque R₁ représente le radical -COOH, les composés sont préparés en protégeant R₁ par un groupe protecteur de type alkyle, allylique, benzylique ou tert-butylique.

Le passage à la forme libre peut être effectué:
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur benzylique, par débenzylation en présence d'hydrogène au moyen d'un catalyseur tel que le palladium sur charbon.
- dans le cas d'un groupement protecteur de type tert-butylique au moyen d'iodure de triméthylsilyle.

La présente invention a également pour objet à titre de médicament les composés de formule (I) telle que définie ci-dessus.

Certains de ces composés présentent une activité dans un test qui consiste à identifier des molécules agonistes des RXRs, tel que décrit dans la demande de brevet français n° 95-07301 déposée le 19 juin 1995 par la Demanderesse. Ce test comprend les étapes suivantes : (i) on applique topiquement sur une partie de la peau d'un mammifère une quantité suffisante d'un composé qui est un ligand actif d'au moins un récepteur de la superfamille des récepteurs stéroïdiens/thyroïdiens, autre qu'un ligand spécifique des récepteurs RXRs, et pouvant s'hétérodimériser avec les RXRs, tel qu'une molécule agoniste des RARs, (ii) on administre par voie systémique ou topique sur cette même partie de la peau du mammifère, avant, pendant ou après l'étape (i), une molécule susceptible de présenter une activité agoniste des RXRs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être augmentée par l'administration par voie systémique ou topique d'une molécule agoniste des RXRs. Certains des composés selon l'invention présentent également une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-altergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose ou l'hypertension ainsi que le diabète non-insulino dépendant,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluéne.

On va maintenant donner, à titre d'illustration et sans aucun caractère limitatif, plusieurs exemples d'obtention de composés actifs de formule (I) selon l'invention, ainsi que diverses formulations concrètes à base de tels composés. Dans ce qui suit ou ce qui précède, les pourcentages sont donnés en poids sauf mention contraire.

### EXEMPLE 1

### Acide 5-(3-tert-butyl-4-méthoxyphényl)-2-thiophèneacrylique.

(a) 5-(3-tert-butyl-4-méthoxyphényl)-2-thiophènecarboxylate de méthyle.
   A une suspension de 300 mg (12 mmoles) de magnésium dans 10 ml de THF, on ajoute goutte à goutte une solution de 2 g (8,2 mmoles) de 3-tert-buty(-4-méthoxybromobenzène. Une fois l'addition terminée, on chauffe à reflux pendant une heure. A température ambiante, on ajoute 1,35 g (9,9 mmoles) de chlorure de zinc anhydre et agite pendant une heure. On ajoute ensuite sucessivement 1,2 g (5,5 mmoles) de 5-bromo-2-thiophènecarboxylate de méthyle et 60 mg (0,12 mmole) du complexe NiCl₂/DPPE et laisse agiter à température ambiante pendant 12 heures. On verse le milieu réactionnel dans l'eau glacée, extrait avec de l'éther éthylique, décante la phase organique, sèche sur sulfate de magnésium, évapore.Le résidu obtenu est chromatographié sur colonne de silice, élué avec un mélange d'hexane et de dichlorométhane (50/50% en volume). Après évaporation des solvants, on recueille 1,56 g (93%) de l'ester méthylique attendu de point de fusion 94-5°C.
(b) 5-(3-tert-butyl-4-méthoxyphényl)-2-thiophèneméthanol.
   Dans un tricot et sous courant d'azote, on introduit 1,5 g (5 mmoles) de l'ester méthylique précédent et 50 ml de THF anhydre. On ajoute 280 mg (7,4 mmoles) d'hydrure double de lithium et d'aluminium et chauffe à reflux pendant quatre heures. On hydrolyse avec une solution de tartrate double de sodium et de potassium, filtre le sel, évapore le filtrat. Le résidu obtenu est purifié par chromatographie sur colonne de silice élué avec un mélange de dichlorométhane et d'hexane (70/30% en volume). Après évaporation des solvants, on recueille 1,26 g (92%) de l'alcoool attendu sous forme d'une huile incolore.
(c) 5-(3-tert-butyl-4-méthoxyphényl)-2-thiophènecarboxaldéhyde.
   Dans un ballon, on introduit 7,15 g (19 mmoles) de pyridinium dichromate et 350 ml de dichlorométhane. A 0°C, on ajoute goutte à goutte une solution de 3,9 g (14 mmoles) de 5-(3-tert-butyl-4-méthoxyphényl)-2-thiophèneméthanol dans 50 ml de dichlorométhane et agite à température ambiante pendant deux heures. Le milieu réactionnel est filtré sur silice, aprè évaporation on recueille 3,26 g (84%) de l'aldéhyde attendu sous forme d'une huile marron.
(d) 5-(3-tert-butyl-4-méthoxyphényl)-2-thiophèneacrylate d'éthyle.
   Dans un tricol et sous courant d'azote, on introduit 200 mg (6,6 mmoles) d'hydrure de sodium (80% dans l'huile) et 50 ml de diméthoxyethane et ajoute goutte à goutte une solution de 1,3 ml (6,6 mmoles) de triéthylphosphonoacétate dans 10 ml de diméthoxyéthane. On agite à température ambiante pendant une heure puis à 0°C on ajoute goutte à goutte une solution de 1,5 g (5,5 mmoles) de 5-(3-tert-butyl-4-méthoxyphényl)-2-thiophènecarboxaldéhyde dans 20 ml de diméthoxyéthane. On agite à température ambiante pendant quatre heures, verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (30170% en volume), on recueille 1,88g (100%) de l'ester éthylique attendu sous forme d'une huile marron.
(e) acide 5-(3-tert-butyl-4-méthoxyphényl)-2-thiophèneacrylique.
   Dans un ballon, on introduit 1,88 g (5,4 mmoles) de l'ester éthylique précédent 20 ml de méthanol et 1,88 g (47 mmoles) d'hydroxyde de sodium et chauffe à reflux pendant quatre heures. On évapore à sec le milieu réactionnel, reprend par l'eau acidifie à pH 1, filtre le solide et sèche. On recristallise le solide obtenu dand l'éthanol, filtre, sèche. On recueille 1,09 g (63%) d'acide 5-(3-tert-butyl-4-méthoxyphényl)-2-thiophèneacrylique de point de fusion 218-9°C.

### EXEMPLE 2

### Acide 5-(3-tert-butyl-4-méthoxyphényl)-2-thiophènepropiolique.

(a) 2'-2'-dibromo-5-(3-tert-butyl-4-méthoxyphényl)-2-thiophèneethylène.
   Dans un ballon on introduit 1,79 g (6,5 mmoles) de 5-(3-tert-butyl-4-méthoxyphényl)-2-thiophènecarboxaldéhyde préparé à l'exemple 1 (c) et 50 ml de dichlorométhane. On ajoute successivement 4,32 g (13 mmoles) de tétrabromure de carbone 3,41 g (13 mmoles) de triphénylphosphine et 850 mg (13 mmoles) de poudre de zinc et agite à température ambiante pendant deux heures. On évapore le milieu réactionnel et purifie le résidu obtenu par chromatographie sur colonne de silice élué avec du dichlorométhane. On recueille 2,5 g (89%) du produit attendu.
(b) 5-(3-tert-butyl-4-méthoxyphényl)-2-thiophèneacétylène.
   Dans un tricol et sous courant d'azote on introduit 2,48 g (5,7 mmoles) de 2'-2'-dibromo-5-(3-tert-butyl-4-méthoxyphényl)-2-thiophèneethylène et 40 ml de THF. A -78°C on ajoute goutte à goutte 5,1 ml (12,7 mmoles) d'une solution de n-butyllithium (2,5 M dans l'hexane) et laisse remonter à température ambiante une heure. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec de l'heptane. On recueille 1,1 g (71%) du dérivé acétylénique attendu sous forme d'une huile jaune.
(c) acide 5-(3-tert-butyl-4-méthoxyphényl)-2-thiophènepropiolique.
   Dans un tricol et sous courant d'azote, on introduit 1,1 g (4 mmoles) du dérivé acétylénique précédent et 20 ml de THF. A -78°C on ajoute goutte à goutte 1,95 ml (4,9 mmoles) de n-butyllithium (2,5 M dans l'hexane) et agite pendant trente minutes. A -78°C, on fait passer un courant de CO₂ pendant quinze minutes et laisse remonter à température ambiante. On verse le milieu réactionnel dans une solution aqueuse de chlorure d'ammonium, extrait avec de l'éther éthylique, décante la phase organique, sèche sur sulfate de magnésium, évapore.Le résidu obtenu est purifié par chromatographie sur colonne de silice élué avec du dichlorométhane. Après évaporation des solvants, on recueille 300 mg (23%) d'acide 5-(3-tert-butyl-4-méthoxyphényl)-2-thiophènepropiolique de point de fusion 124-6°C.

### EXEMPLE 3

### Acide 2-(3-tert-butyl-4-méthoxyphényl)-4-thiophèneacrylique.

(a) acide 3-tert-butyl-4-méthoxyphénylboronique.
   Dans un tricol et sous courant d'azote, on introduit 4 g (16,5 mmoles) de 3-tert-butyl-4-méthoxybromobenzène et 50 ml de THF. A -78°C, on ajoute goutte à goutte 7,9 ml (19,8 mmoles) de n-butyllithium (2,5M dans l'hexane) et agite 15', à cette même température on ajoute 5,6 ml (49,5 mmoles) de triméthylborate et agite pendant 2 heures. A -50°C on ajoute 20 ml d'acide chlorhydrique (1N) et laisse remonter à trempérature ambiante. On extrait le milieu réactionnel avec de l'éther éthylique, décante la phase organique, sèche sur sulfate de magnésium, évapore. On recueille 3,79 g (100%) d'acide boronique attendu qui est utilisé telquel pour la suite de la synthèse.
(b) 2-(3-tert-butyl-4-méthoxyphényl)-4-thiophènecarboxylate d'éthyle.
   Dans un tricol et sous courant d'azote, on introduit 260 mg (0,5 mmole) de tétrakis(triphénylphosphine)palladium(0) 50 ml de toluène et 2,59 g (10,9 mmoles) de 2-bromo-4-thiophènecarboxylate d'éthyle et agite à température ambiante 20'. On ajoute ensuite 3,7 g (16,5 mmoles) d'acide 3-tert-butyl-4-méthoxyphénylboronique 11 ml d'une solution aqueuse de carbonate de sodium (2N) et chauffe à reflux pendant 8 heures. On évapore à sec le milieu réactionnel, reprend par l'eau et l'éther éthylique, décante la phase organique, sèche sur sulfate de magnésium, évapore. Le résidu est purifié par chromatographie sur colonne de silice élué avec un mélange d'acétate d'éthyle et d'heptane (10/90% en volume). On obtient 3,53 g (69%) de 2-(3-tert-butyl-4-méthoxyphényl)-4-thiophènecarboxylate d'éthyle.
(c) 2-(3-tert-butyl-4-méthoxyphényl)-4-thiophèneméthanol.
   De manière analogue à l'exemple 1(b) à partir de 3,5 g (11 mmoles) de 2-(3-tert-butyl-4-méthoxyphényl)-4-thiophènecarboxylate d'éthyle, on obtient 3,2 g (100%) de l'alcool attendu sous forme d'une huile marron.
(d) 2-(3-tert-butyl-4-méthoxyphényl)-4-thiophènecarboxaldéhyde.
   De manière analogue à l'exemple 1(c) à partir de 3,2 g (11 mmoles) de l'alcool précédent, on obtient 2,3 g (76%) de 2-(3-tert-butyl-4-méthoxyphényl)-4-thiophènecarboxaldéhyde sous forme d'une huile marron.
(e) 2-(3-tert-butyl-4-méthoxyphényl)-4-thiophèneacrylate d'éthyle.
   De manière analogue à l'exemple 1(d) par réaction de 1,3 g (4,7 mmoles) de 2-(3-tert-butyl-4-méthoxyphényl)-4-thiophènecarboxaldéhyde avec 1,28 g (5,7 mmoles) de triéthylphosphonoacétate, on obtient 1,1 g (67%) de l'ester éthylique attendu de point de fusion 119-20°C.
(f) acide 2-(3-tert-butyl-4-méthoxyphényl)-4-thiophèneacrylique.
   De manière analogue à l'exemple 1(e) à partir de 1,1 g (3,2 mmoles) de l'ester éthylique précédent, on obtient 750 mg (74%) d'acide 2-(3-tert-butyl-4-méthoxyphényl)-4-thiophèneacrylique de point de fusion 197-8°C.

### EXEMPLE 4

### Acide 4-(3-tert-butyl-4-méthoxyphényl)-2-thiophéneacrylique.

(a) 4-(3-tert-butyl-4-méthoxyphényl)-2-thiophènecarboxaldéhyde.
   De manière analogue à l'exemple 3(b) par réaction de 2,68 g (12,3 mmoles) d'acide 3-tert-butyl-4-méthoxyphénylboronique avec 1,55 g (2,12 mmoles) de 4-bromo-2-thiophènecarboxaldéhyde, on obtient 2,13 g (95%) de 4-(3-tert-butyl-4-méthoxyphényl)-2-thiophènecarboxaldéhyde sous forme d'une huile jaune.
(b) 4-(3-tert-butyl-4-méthoxyphényl)-2-thiophèneacrylate d'éthyle.
   De manière analogue à l'exemple 1(d) par réaction de 1,2 g (4,3 mmoles) de 4-(3-tert-butyl-4-méthoxyphényl)-2-thiophènecarboxaldéhyde avec 1,17 g (5,2 mmoles) de triéthylphosphonoacétate, on obtient 1,55 g (100%) de l'ester éthylique attendu sous forme d'une huile.
(c) acide 4-(3-tert-butyl-4-méthoxyphényl)-2-thiophèneacrylique.
   De manière analogue à l'exemple 1(e) à partir de 1,55 g (4,5 mmoles) de l'ester éthylique précédent, on obtient 1,14 g (88%) d'acide 4-(3-tert-butyl-4-méthoxyphényl)-2-thiophèneacrylique de point de fusion 206-7°C.

### EXEMPLE 5

### Acide 5-(3,5,5,8,8-pentaméthyl-5,6,7,8-tétrahydro-2-naphtyl)-2-thiophène acrylique.

(a) acide 3,5,5,8,8-pentaméthyl-5,6,7,8-tétrahydro-2-naphtylboronique.
   De manière analogue à l'exemple 3(a) à partir de 5 g (17,8 mmoles) de 3,5,5,8,8-pentaméthyl-5,6,7,8-tétrahydro-2-bromonaphtalène, on obtient 4,22 g (100%) d'acide boronique.
(b) 5-(3,5,5,8,8-pentaméthyl-5,6,7,8-tétrahydro-2-naphtyl)-2-thiophène carboxaldéhyde.
   De manière analogue à l'exemple 3(b) par réaction de 4,2 g (17 mmoles) d'acide 3,5,5,8,8-pentaméthyl-5,6,7,8-tétrahydro-2-naphtylboronique avec 2,17 g (11,3 mmoles) de 5-bromo-2-thiophènecarboxaldéhyde, on obtient 2,1 g (60%) de l'aldéhyde attendu de point de fusion 130-5°C.
(c) 5-(3,5,5,8,8-pentaméthyl-5,6,7,8-tétrahydro-2-naphtyl)-2-thiophèneacrylate d'éthyle.
   De manière analogue à l'exemple 1(d) par réaction de 2 g (6,4 mmoles) de 5-(3,5,5,8,8-pentaméthyl-5,6,7,8-tétrahydro-2-naphtyl)-2-thiophènecarboxaldéhyde avec 1,73 g (7,7 mmoles) de triéthylphosphonoacétate, on obtient 2,02 g (82%) de l'ester éthylique attendu.
(e) acide 5-(3,5,5,8,8-pentaméthyl-5,6,7,8-tétrahydro-2-naphtyl)-2-thiophène acrylique.
   De manière analogue à l'exemple 1(e) à partir de 2 g (5,2 mmoles) de l'ester éthylique précédent, on obtient 1,79 g (96%) d'acide 5-(3,5,5,8,8-pentaméthyl-5,6,7,8-tétrahydro-2-naphtyl)-2-thiophèneacrylique de point de fusion 175-7°C.

### EXEMPLE 6

### Acide 4-(3,5,5,8,8-pentaméthyl-5,6,7,8-tétrahydro-2-naphtyl)-2-thiophène acrylique.

(a) 4-(3,5,5,8,8-pentaméthyl-5,6,7,8-tétrahydro-2-naphtyl)-2-thiophène carboxaldéhyde.
   De manière analogue à l'exemple 3(b) par réaction de 4,2 g (17 mmoles) d'acide 3,5,5,8,8-pentaméthyl-5,6,7,8-tétrahydro-2-naphtylboronique avec 2,17 g (11,3 mmoles) de 4-bromo-2-thiophène carboxaldéhyde, on obtient 2,75 g (78%) de l'aldéhyde attendu de point de fusion 144-6°C.
(b) 4-(3,5,5,8,8-pentaméthyl-5,6,7,8-tétrahydro-2-naphtyl)-2-thiophèneacrylate d'éthyle.
   De manière analogue à l'exemple 1(d) par réaction de 2,7 g (8,6 mmoles) de 4-(3,5,5,8,8-pentaméthyl-5,6,7,8-tétrahydro-2-naphtyl)-2-thiophènecarboxaldéhyde avec 2,1 ml (10,4 mmoles) de triéthylphosphonoacétate, on obtient 2,76 g (84%) de l'ester éthylique attendu.
(c) acide 4-(3,5,5,8,8-pentaméthy)-5,6,7,8-tétrahydro-2-naphtyl)-2-thiophène acrylique.
   De manière analogue à l'exemple 1(e) à partir de 2,7 g (7,1 mmoles) de l'ester éthylique précédent, on obtient 2,5 g (98%) d'acide 4-(3,5,5,8,8-pentaméthyl-5,6,7,8-tétrahydro-2-naphtyl)-2-thiophèneacrylique de point de fusion 215-20°C.

### EXEMPLE 7

### Acide 4-(5,6,7,8-tétrahydro-5,5,8,8-tétraméthyl-2-naphtyl)-2-thiophène acrylique.

(a) acide 5,6,7,8-tétrahydro-5,5,8,8-tétraméthyl-2-naphtylboronique.
   De manière analogue à l'exemple 3(a) à partir de 5 g (18,7 mmoles) de 5,6,7,8-tétrahydro-5,5,8,8-tétraméthyl-2-bromonaphtalène, on obtient 4,3 g (100%) d'acide boronique attendu.
(b) 4-(5,6,7,8-tétrahydro-5,5,8,8-tétraméthyl-2-naphtyl)-2-thiophènecarboxaldéhyde.
   De manière analogue à l'exemple 3(b) par réaction de 4,3 g (18,7 mmoles) d'acide 5,6,7,8-tétrahydro-5,5,8,8-tétraméthyl-2-naphtylboronique avec 2,36 g (12,3 mmoles) de 4-bromo-2-thiophènecarboxaldéhyde, on obtient 2,3 g (63%) du dérivé aldéhydique attendu de point de fusion 84-5°C.
(c) 4-(5,6,7,8-tétrahydro-5,5,8,8-tétraméthyl-2-naphtyl)-2-thiophèneacrylate d'éthyle.
   De manière analogue à l'exemple 1(d) par réaction de 2,28 g (8,3 mmoles) de (5,6,7,8-tétrahydro-5,5,8,8-tétraméthyl-2-naphtyl)-2-thiophènecarboxaldéhyde avec 2 ml (9,9 mmoles) de triéthylphosphonoacétate, on obtient 810 mg (26%) de l'ester éthylique attendu de point de fusion 82-4°C.
(d) acide 4-(5,6,7,8-tétrahydro-5,5,8,8-tétraméthyl-2-naphtyl)-2-thiophèneacrylique.
   De manière analogue à l'exemple 1(e) à partir de 810 mg (2,2 mmoles) de l'ester éthylique précédent, on obtient 720 mg (96%) d'acide 4-(5,6,7,8-tétrahydro-5,5,8,8-tétraméthyl-2-naphtyl)-2-thiophèneacrylique de point de fusion 182-5°C.

### EXEMPLE 8

### Acide 5-(3,5,5,8,8-pentaméthyl-5,6,7,8-tétrahydro-2-naphtyl)-2-thiophène propiolique.

(a) 2'-2'-dibromo-5-(3,5,5,8,8-pentaméthyl-5,6,7,8-tétrahydro-2-naphtyl)-2-thiophèneethylène.
   De manière analogue à l'exemple 2(a) à partir de 3 g (9,6 mmoles) de 5-(3,5,5,8,8-pentaméthyl-5,6,7,8-tétrahydro-2-naphtyl)-2-thiophènecarboxaldéhyde, on obtient 4,56 g (100%) de 2'-2'-dibromo-5-(3,5,5,8,8-pentaméthyl-5,6,7,8-tétrahydro-2-naphtyl)-2-thiophèneethylène.
(b) 5-(3,5,5,8,8-pentaméthyl-5,6,7,8-tétrahydro-2-naphtyl)-2-thiophèneacétylène.
   De manière analogue à l'exemple 2(b) à partir de 4,5 g (9,6 mmoles) de 2'-2'-dibromo-5-(3,5,5,8,8-pentaméthyl-5,6,7,8-tétrahydro-2-naphtyl)-2-thiophène ethylène, on obtient 1,42 g (48%) de 5-(3,5,5,8,8-pentaméthyl-5,6,7,8-tétrahydro-2-naphtyl)-2-thiophèneacétylène.
(c) acide 5-(3,5,5,8,8-pentaméthyl-5,6,7,8-tétrahydro-2-naphtyl)-2-thiophène propiolique.
   De manière analogue à l'exemple 2(c) à partir de 1,4 g (4,5 mmoles) du dérivé acétylénique précédent, on obtient 800 mg (51%) d'acide 5-(3,5,5,8,8-pentaméthyl-5,6,7,8-tétrahydro-2-naphtyl)-2-thiophène propiolique de point de fusion 138-40°C.

### EXEMPLE 9

### Acide 3-(3,5,5,8,8-pentaméthyl-5,6,7,8-tétrahydro-2-naphtyl)phenyl propiolique.

(a) acide 3,5,5,8,8-pentaméthyl-5,6,7,8-tétrahydro-2-naphtylboronique.
   Dans un réacteur de deux litres et sous courant d'azote, on introduit 100 g (0,356 mole) de 2-bromo-3,5,5,8,8-pentaméthyl-5,6,7,8-tétrahydro-2-naphtalène 1 litre de THF et refroidit à -60°C. On ajoute goutte à goutte 157 ml (0,392 mole) de n-butyllithium (2,5 M dans l'hexane) et agite une heure. A -70°C on ajoute goutte à goutte 121 ml (1,07 mole) de borate de triméthyle et agite pendant une heure. A -35°C, on ajoute 500 ml d'acide chlorhydrique (1N) et laisse remonter à température ambiante. On extrait le milieu réactionnel avec de l'acétate d'éthyle, décante la phase organique, lave deux fois avec 500 ml d'acide chlorhydrique (1N), sèche sur sulfate de magnésium, évapore. On recueille 83 g (95%) de l'acide boronique attendu .
(b) 3-(3,5,5,8,8-pentaméthyl-5,6,7,8-tétrahydro-2-naphtyl)benzaldehyde.
   Dans un tricol et sous courant d'azote, on introduit 700 ml de DME 2,4 g (2 mmoles) de tetrakistriphenylphosphinepalladium(0) 8,44 g (45,6 mmoles) de 3-bromobenzaldéhyde et agite 10 minutes. On ajoute ensuite une solution de 17 g (69,1 mmoles) d'acide 3,5,5,8,8-pentaméthyl-5,6,7,8-tétrahydro-2-naphtylboronique dans 25 ml d'éthanol puis 46 ml (91 mmoles) d'une solution de carbonate de potassium (2M) et chauffe à reflux pendant quatre heures. On refroidit le milieu réactionnel, filtre le solide, le lave à l'eau bicarbonatée puis à l'acétate d'éthyle. On recristallise le solide obtenu dans l'éthanol, recueille 7 g (50%) de l'aldéhyde attendu de point de fusion 104-5°C.
(c) 2'-2'-dibromo-3-(3,5,5,8,8-pentaméthyl-5,6,7,8-tétrahydro-2-naphtyl)phenyl ethylène.
   De manière analogue à l'exemple 2(a) à partir de 2 g (6,5 mmoles) de l'aldéhyde précédent, on obtient 1,96 g (65%) de produit attendu sous forme d'une huile incolore.
(c) 3-(3,5,5,8,8-pentaméthyl-5,6,7,8-tétrahydro-2-naphtyl)phenylacetylène.
   De manière analogue à l'exemple 2(b) à partir de 1,96 g (4,23 mmoles) de 2'-2'-dibromo-3-(3,5,5,8,8-pentaméthyl-5,6,7,8-tétrahydro-2-naphtyl)phenylethylène, on obtient 1,29 g (99%) d'acétylénique attendu sous forme d'une huile légèrement jaune.
(d) acide 3-(3,5,5,8,8-pentaméthyl-5,6,7,8-tétrahydro-2-naphtyl)phenylpropiolique.
   De manière analogue à l'exemple 2(c) à partir de 1,17 g (3,9 mmoles) du dérivé acétylénique précédent, on obtient 900 mg (67%) d'acide 3-(3,5,5,8,8-pentaméthyl-5,6,7,8-tétrahydro-2-naphtyl)phenylpropiolique de point de fusion 180-1°C.

### EXEMPLE 10

### Acide N-méthyl-4-(5,6,7,8-tétrahydro-5,5,8,8-tétraméthyl-2-naphtyl)-2-pyrroleacrylique.

(a) 4-(5,6,7,8-tétrahydro-5,5,8,8-tétraméthyl-2-naphtyl)-2-pyrrolecarboxaldéhyde.
   De manière analogue à l'exemple 3(b) par réaction de 5,9 g (25,6 mmoles) d'acide 5,6,7,8-tétrahydro-5,5,8,8-tétraméthyl-2-naphtylboronique avec 3,7 g (21,3 mmoles) de 4-bromo-2-pyrrolecarboxaldéhyde, on obtient 1,3 g (21,6%) de produit attendu de point de fusion 211-2°C.
(b) N-méthyl-4-(5,6,7,8-tétrahydro-5,5,8,8-tétraméthyl-2-naphtyl)-2-pyrrole carboxaldéhyde.
   Dans un tricol et sous courant d'azote, on introduit 1,3 g (4,6 mmoles) de 4-(5,6,7,8-tétrahydro-5,5,8,8-tétraméthyl-2-naphtyl)-2-pyrrolecarboxaldéhyde et 50 ml de THF. On ajoute par petites quantités 300 mg (10 mmoles) d'hydrure de sodium (80% dans l'huile) et agite jusqu'à cessation du dégagement gazeux. On ajoute ensuite 640 µl (10 mmoles) d'iodomethane et agite à température ambiante pendant une heure. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange de dichlorométhane et d'heptane (70-30). Après évaporation des solvants, on recueille 600 mg (44%) du produit attendu
(c) N-méthyl-4-(5,6,7,8-tétrahydro-5,5,8,8-tétraméthyl-2-naphtyl)-2-pyrroleacrylate d'éthyle.
   De manière analogue à l'exemple 1(d) par réaction de 480 mg (1,3 mmoles) de N-méthyl-4-(5,6,7,8-tétrahydro-5,5,8,8-tétraméthyl-2-naphtyl)-2-pyrrole carboxaldéhyde avec 400 µl (152 mmoles) de triéthylphosphonoacetate, on obtient 350 mg de l'ester éthylique attendu sous forme d'une huile.
(d) acide N-méthyl-4-(5,6,7,8-tétrahydro-5,5,8,8-tétraméthyl-2-naphtyl)-2-pyrrole acrylique.
   De manière analogue à l'exemple 1(e) à partir de 350 mg (0,94 mmole) de l'ester éthylique précédent, on obtient 170 mg (23%) d'acide N-méthyl-4-(5,6,7,8-tétrahydro-5,5,8,8-tétraméthyl-2-naphtyl)-2-pyrroleacrylique de point de fusion 185-6°C.

### EXEMPLE 11

### Acide 3-(5,6,7,8-tétrahydro-5,5,8,8-tétraméthyl-2-naphtyl)phenylacrylique.

(a) 3-(5,6,7,8-tétrahydro-5,5,8,8-tétraméthyl-2-naphtyl)phenylcarboxaldéhyde.
   De manière analogue à l'exemple 3(b) par réaction de 6,43 g (27,7 mmoles) d'acide 5,6,7,8-tétrahydro-5,5,8,8-tétraméthyl-2-naphtylboronique avec 2,7 ml (23,1 mmoles) de 4-bromobenzaldehyde, on obtient 2,05 g (24%) de 3-(5,6,7,8-tétrahydro-5,5,8,8-tétraméthyl-2-naphtyl)phenylcarboxaldéhyde sous forme d'une huile légèrement jaune.
(b) 3-(5,6,7,8-tétrahydro-5,5,8,8-tétraméthyl-2-naphtyl)phenylacrylate d'éthyle.
   De manière analogue à l'exemple 1(d) par réaction de 800 mg (2,7 mmoles) de 3-(5,6,7,8-tétrahydro-5,5,8,8-tétraméthyl-2-naphtyl)phenylcarboxaldéhyde avec 650 ml (3,3 mmoles) de triéthylphosphonoacétate, on obtient 900 mg (91%) de l'ester éthylique attendu sous forme d'une huile incolore.
(c) acide 3-(5,6,7,8-tétrahydro-5,5,8,8-tétraméthyl-2-naphtyl)phenylacrylique.
   De manière analogue à l'exemple 1(e) à partir de 1,22 g (2,7 mmoles) de l'ester éthylique précédent, on obtient 380 mg (41%) d'acide 3-(5,6,7,8-tétrahydro-5,5,8,8-tétraméthyl-2-naphtyl)phenylacrylique de point de fusion 210-1°C.

### EXEMPLE 12

### Acide N-méthyl-4-(3,5,5,8,8-pentaméthyl-5,6,7,8-tétrahydro-2-naphtyl)-2-pyrroleacrylique.

(a) N-méthyl-4-bromo-2-pyrrolecarboxaldéhyde.
   De manière analogue à l'exemple 10 (b) par réaction de 4 g (23 mmoles) de 4-bromo-2-pyrrolecarboxaldéhyde avec 1,7 ml (27,6 mmoles) d'iodométhane, on obtient 2,3 g (50%) de produit attendu de point de fusion 123-4°C.
(b) N-méthyl-4-(3,5,5,8,8-pentaméthyl-5,6,7,8-tétrahydro-2-naphtyl)-2-pyrrole carboxaldehyde.
   De maniére analogue à l'exemple 3(b) par réaction de 3 g (12,1 mmoles) d'acide 3,5,5,8,8-pentaméthyl-5,6,7,8-tétrahydro-2-naphtylboronique avec 1,9 g (10,1 mmoles) de N-méthyl-4-bromo-2-pyrrolecarboxaldéhyde, on obtient 1,85 g (59%) de produit attendu sous forme d'une huile légèrement jaune.
(c) N-méthyl-4-(3,5,5,8,8-pentaméthyl-5,6,7,8-tétrahydro-2-naphtyl)-2-pyrroleacrylate d'éthyle.
   De manière analogue à l'exemple 1(d) par réaction de 1,85 g (6 mmoles) de N-méthyl-4-(3,5,5,8,8-pentaméthyl-5,6,7,8-tétrahydro-2-naphtyl)-2-pyrrole carboxaldehyde avec 1,4 ml (7,2 mmoles) de triéthylphosphonoacétate, on obtient 2,1 g (92%) de l'ester éthylique attendu sous forme d'une huile orangée.
(d) acide N-méthyl-4-(3,5,5,8,8-pentaméthyl-5,6,7,8-tétrahydro-2-naphtyl)-2-pyrroleacrylique.
   De manière analogue à l'exemple 1(e) à partir de 2 g (5,3 mmoles) de l'ester éthylique précédent, on obtient 730 mg (39.5%) d'acide N-méthyl-4-(3,5,5,8,8-pentaméthyl-5,6,7,8-tétrahydro-2-naphtyl)-2-pyrroleacrylique de point de fusion 185-6°C.

### EXEMPLE 13

### Acide 4-(3,5,5,8,8-pentaméthyl-5,6,7,8-tétrahydro-2-naphtyl)-2-pyrrole acrylique.

(a) 4-(3,5,5,8,8-pentaméthyl-5,6,7,8-tétrahydro-2-naphtyl)-2-pyrrolecarboxaldehyde.
   De manière analogue à l'exemple 3(b) par réaction de 2,47 g (10 mmoles) d'acide 3,5,5,8,8-pentaméthyl-5,6,7,8-tétrahydro-2-naphtylboronique avec 1,5 g (8,4 mmoles) de 4-bromo-2-pyrrolecarboxaldehyde, on obtient 950 mg (38,5%) de l'aldéhyde attendu de point de fusion 128-9°C.
(b) 4-(3,5,5,8,8-pentaméthyl-5,6,7,8-tétrahydro-2-naphtyl)-2-pyrroleacrylate d'éthyle.
   De maniére analogue à l'exemple 1(d) par réaction de 500 mg (1,7 mmoles) de 4-(3,5,5,8,8-pentaméthyl-5,6,7,8-tétrahydro-2-naphtyl)-2-pyrrolecarboxaldehyde avec 400 µl (2 mmoles) de triéthylphosphonoacétate, on obtient 570 mg (92%) de l'ester éthylique attendu
(c) acide 4-(3,5,5,8,8-pentaméthyl-5,6,7,8-tétrahydro-2-naphtyl)-2-pyrroleacrylique.
   De manière analogue à l'exemple 1(e) à partir de 570 mg (1,9 mmoles) de 4-(3,5,5,8,8-pentaméthyl-5,6,7,8-tétrahydro-2-naphtyl)-2-pyrrole acrylate d'éthyle, on obtient 240 mg (37%) d'acide 4-(3,5,5,8,8-pentaméthyl-5,6,7,8-tétrahydro-2-naphtyl)-2-pyrroleacrylique de point de fusion 245-6°C.

### EXEMPLE 14

### Acide 3-(3,5,5,8,8-pentaméthyl-5,6,7,8-tétrahydro-2-naphtyl)phenylacrylique.

De manière analogue à l'exemple 9(b) par réaction de 73,4 g (0,30 mole) d'acide 3,5,5,8,8-pentaméthyl-5,6,7,8-tétrahydro-2-naphtylboronique avec 44,7 g (0,20 mole) d'acide 4-bromophenylacrylique, on obtient après recristallisation dans l'éthanol 48 g (61 %) d'acide 3-(3,5,5,8,8-pentaméthyl-5,6,7,8-tétrahydro-2-naphtyl)phenylacrylique de point de fusion 207-8°C.

### EXEMPLE 15

### Acide 3-(5,6,7,8-tétrahydro-5,5,8,8-tétraméthyl2-naphtyl)phenylpropiolique.

(a) 2'-2'-dibromo-3-(5,6,7,8-tétrahydro-5,5,8,8-tétraméthyl-2-naphtyl)phenyléthylène.
   De manière analogue à l'exemple 2(a) à partir de 2,05 g (7 mmoles) de 3-(5,6,7,8-tétrahydro-5,5,8,8-tétraméthyl-2-naphtyl)benzaldehyde {[préparé à l'exemple 11(a)], on obtient 1,07 (35%) de produit attendu sous forme d'une huile.
(b) acide 3-(5,6,7,8-tétrahydro-5,5,8,8-tétraméthyl-2-naphtyl)phenylpropiolique.
   Dans un tricol et sous courant d'azote, on introduit 900 mg (2 mmoles) de 2'-2'-dibromo-3-(5,6,7,8-tétrahydro-5,5,8,8-tétraméthyl2-naphtyl)phenyléthylène et 40 ml de THF. A -50°C on ajoute goutte à goutte 2,2 ml (5,2 mmoles) d'une solution de n-butyllithium (2,5M dans l'hexane) et laisse remonter à température ambiante. A 0°C, on introduit du CO₂ pendant 20 minutes et agite à température ambiante pendant une heure. On verse le milieu réactionnel dans une solution saturée de chlorure d'ammonium, ajuste à pH 1 avec de l'acide chlorhydrique, extrait avec de l'acétate d'éthyle, décante la phase organique, sèche sur sulfate de magnésium, évapore. Le résidu obtenu est purifié par chromatographie sur colonne de silice élué avec un mélange de dichlorométhane et de méthanol (95-5). Après évaporation des solvants, on recueille 80 mg (12%) d'acide 3-(5,6,7,8-tétranydro-5,5,8,8-tétraméthyl-2-naphtyl)phenylpropiolique de point de fusion 164-5°C.

### EXEMPLE 16

### Acide 2-methoxy-5-(3,5,5,8,8-pentaméthyl-5,6,7,8-tétrahydro-2-naphtyl) phenylacrylique.

(a) 2-hydroxy-5-(3,5,5,8,8-pentaméthyl-5,6,7,8-tétrahydro-2-naphtyl)benzaldéhyde.
   De manière analogue à l'exemple 9(b) par réaction de 15 g (61 mmoles) d'acide 3,5,5,8,8-pentaméthyl-5,6,7,8-tétrahydro-2-naphtylboronique avec 8,16 g (41 mmoles) de 5-bromo-2-hydroxybenzaldéhyde, on obtient 11,7 g (89%) de 2-hydroxy-5-(3,5,5,8,8-pentaméthyl-5,6,7,8-tétrahydro-2-naphtyl)benzaldéhyde de point de fusion 138-9°C.
(b) 2-methoxy-5-(3,5,5,8,8-pentaméthyl-5,6,7,8-tétrahydro-2-naphtyl)benzaldéhyde.
   De manière analogue à l'exemple 10(b) par réaction de 2 g (6,2 mmoles) de 2-hydroxy-5-(3,5,5,8,8-pentaméthyl-5,6,7,8-tétrahydro-2-naphtyl)benzaldéhyde avec 425 µl (6,8 mmoles) d'iodomethane, on obtient 1,68 g (88%) du produit attendu.
(c) 2-methoxy-5-(3,5,5,8,8-pentaméthyl-5,6,7,8-tétrahydro-2-naphtyl)phenylacrylate d'éthyle.
   De manière analogue à l'exemple 1(d) par réaction de 1,65 g (5 mmoles) de 2-methoxy-5-(3,5,5,8,8-pentaméthyl-5,6,7,8-tétrahydro-2-naphtyl)benzaldéhyde avec 1,68 g (7,5 mmoles) de triéthylphosphonoacétate, on obtient 1,7 g (83%) de l'ester éthylique attendu sous forme d'une huile.
(d) acide 2-methoxy-5-(3,5,5,8,8-pentaméthyl-5,6,7,8-tétrahydro-2-naphtyl) phenylacrylique.
   De manière analogue à l'exemple 1(e) à partir de 1,6 g (3,9 mmoles) de l'ester ethylique précédent, on obtient 1,4 g (93%) d'acide 2-methoxy-5-(3,5,5,8,8-pentaméthyl-5,6,7,8-tétrahydro-2-naphtyl)phenylacrylique de point de fusion 181-2°C.

### EXEMPLE 17

### Acide 2-propyloxy-5-(3,5,5,8,8-pentaméthyl-5,6,7,8-tétrahydro-2-naphtyl) phenylacrylique.

(a) 2-propyloxy-5-(3,5,5,8,8-pentaméthyl-5,6,7,8-tétrahydro-2-naphtyl)benzaldéhyde.
   De manière analogue à l'exemple 10(b) par réaction de 2 g (6,2 mmoles) de 2-hydroxy-5-(3,5,5,8,8-pentaméthyl-5,6,7,8-tétrahydro-2-naphtyl)benzaldéhyde [préparé à l'exemple 16(a)] avec 670 µl (6,8 mmoles) de 1-iodopropane, on obtient 2,2 g (88%) du produit attendu sous forme d'une huile transparente.
(b) 2-propyloxy-5-(3,5,5,8,8-pentaméthyl-5,6,7,8-tétrahydro-2-naphtyl)phenyl acrylate d'éthyle.
   De manière analogue à l'exemple 1(d) par réaction de 2,18 g (6 mmoles) de 2-propyloxy-5-(3,5,5,8,8-pentaméthyl-5,6,7,8-tétrahydro-2-naphtyl)benzaldéhyde avec 2,03 g (9 mmoles) de triéthylphosphonoacétate, on obtient 2,13 g (82%) de l'ester éthylique attendu sous forme d'une huile jaune.
(c) acide 2-propyloxy-5-(3,5,5,8,8-pentaméthyl-5,6,7,8-tétrahydro-2-naphtyl) phenyfacrylique.
   De manière analogue à l'exemple 1(e) à partir de 2,1 g (4,8 mmoles) de l'ester éthylique précédent, on obtient 1,68 g (86%) d'acide 2-propyloxy-5-(3,5,5,8,8-pentaméthyl-5,6,7,8-tétrahydro-2-naphtyl)phenylacrylique de point de fusion 125-6°C.

### EXEMPLE 18

### Acide 2-heptyloxy-5-(3,5,5,8,8-pentaméthyl-5,6,7,8-tétrahydro-2-naphtyl) phenylacrylique.

(a) 2-heptyloxy-5-(3,5,5,8,8-pentaméthyl-5,6,7,8-tétrahydro-2-naphtyl)benzaldéhyde.
   De manière analogue à l'exemple 10(b) par réaction de 2 g (9,3 mmoles) de 2-hydroxy-5-(3,5,5,8, 8-pentaméthyl-5,6,7,8-tétrahydro-2-naphtyl)benzaldéhyde [préparé à l'exemple 16(a)] avec1,1 ml (6,8 mmoles) de 1-bronoheptane, on obtient 1,88 g (72%) du produit attendu sous forme d'une huile jaune.
(b) 2-heptyloxy-5-(3,5,5,8,8-pentaméthyl-5,6,7,8-tétrahydro-2-naphtyl)phenyl acrylate d'éthyle.
   De manière analogue à l'exemple 1(d) par réaction de 1,78 g (4,2 mmoles) de 2-heptyloxy-5-(3,5,5,8,8-pentaméthyl-5,6,7,8-tétrahydro-2-naphtyl)benzaldéhyde avec 1,44 g (6,3 mmoles) de triéthylphosphonoacétate, on obtient 1,89 g (90%) de l'ester éthylique attendu sous forme d'une huile jaune.
(c) acide 2-heptyloxy-5-(3,5,5,8,8-pentaméthyl-5,6,7,8-tétrahydro-2-naphtyl) phenylacrylique.
   De manière analogue à l'exemple 1(e) à partir de 1,89 g (3,9 mmoles) de l'ester éthylique précédent, on obtient 1,2 g (67%) d'acide 2-heptyloxy-5-(3,5,5,8,8-pentaméthy)-5,6,7,8-tétrahydro-2-naphtyl) phenylacrylique de point de fusion 137-8°C.

### EXEMPLE 19

### Acide 2-methoxymethoxy-5-(3,5,5,8,8-pentaméthyl-5,6,7,8-tétrahydro-2-naphtyl) phenylacrylique.

(a) 2-methoxymethoxy-5-(3,5,5,8,8-pentaméthyl-5,6,7,8-tétrahydro-2-naphtyl) benzaldéhyde.
   De manière analogue à l'exemple 10(b) par réaction de 3 g (9,3 mmoles) de 2-hydroxy-5-(3,5,5,8,8-pentaméthyl-5,6,7,8-tétrahydro-2-naphtyl)benzaldéhyde [préparé à l'exemple 16(a)] avec 777 µl (10.2 mmoles) de chlorure de methoxymethyle, on obtient 3,5 g (100%) du produit attendu sous forme d'une huile.
(b) 2-methoxymethoxy-5-(3,5,5,8,8-pentaméthyl-5,6,7,8-tétrahydro-2-naphtyl) phenylacrylate d'éthyle.
   De maniére analogue à l'exemple 1(d) par réaction de 3,4 g (9,3 mmoles) de 2-methoxymethoxy-5-(3,5,5,8,8-pentaméthyl-5,6,7,8-tétrahydro-2-naphtyl) benzaldéhyde avec 4,16 g (18,6 mmoles) de triéthylphosphonoacétate, on obtient 3,5 g (86%) de l'ester éthylique attendu de point de fusion 100-1°C.
(c) acide 2-methoxymethoxy-5-(3,5,5,8,8-pentaméthyl-5,6,7,8-tétrahydro-2-naphtyl) phenylacrylique.
   De manière analogue à l'exemple 1(e) à partir de 1,5 g (3,4 mmoles) de l'ester éthylique précédent, on obtient 1,2 g (86%) d'acide 2-methoxymethoxy-5-(3,5,5,8,8-pentaméthyl-5,6,7,8-tétrahydro-2-naphtyl)phenylacrylique de point de fusion 191-2°C.

### EXEMPLE 20

### Acide 2-hydroxy-5-(3,5,5,8,8-pentaméthyl-5,6,7,8-tétrahydro-2-naphtyl) phenylacrylique.

(a) 2-hydroy-5-(3,5,5,8,8-pentaméthyl-5,6,7,8-tétrahydro-2-naphtyl)phenylacrylate de méthyle.
   Dans un ballon, on introduit 1,9 g (4,35 mmoles) d'acide 2-methoxymethoxy-5-(3,5,5,8,8-pentaméthyl-5,6,7,8-tétrahydro-2-naphtyl)phenylacrylique 20 ml de méthanol et 30 ml de THF. On ajoute 2,8 ml d'acide sulfurique concentré et agite à température ambiante pendant 12 heures. On verse le milieu réactionnel dans l'eau, extrait avec de l'éther éthylique, décante la phase organique, lave à l'eau, sèche sur sulfate de magnésium, évapore. On recueille 1,63 g (95%) de 2-hydroy-5-(3,5,5,8,8-pentaméthyl-5,6,7,8-tétrahydro-2-naphtyl)phenylacrylate de méthyle.
(b) acide 2-hydroxy-5-(3,5,5,8,8-pentaméthyl-5,6,7,8-tétrahydro-2-naphtyl) phenylacrylique.
   De manière analogue à l'exemple 1(e) à partir de 1,63 g (4,25 mmoles) de l'ester éthylique précédent, on obtient 1,3 g (85%) d'acide 2-hydroxy-5-(3,5,5,8,8-pentaméthyl-5,6,7,8-tétrahydro-2-naphtyl)phenylacrylique de point de fusion 204-5°C.

### EXEMPLE 21

### Acide 3-(3-methoxy-5,6,7,8-tétrahydro-5,5,8,8-tétraméthyl-2-naphtyl) phenylacrylique.

(a) 3-bromo-2-methoxy-5,6,7,8-tétrahydro-5,5,8,8-tétraméthylnaphtalene.
   Dans un tricot et sous courant d'azote, on introduit 7 g (24,7 mmoles) de 3-bromo-2-hydroxy-5,6,7,8-tétrahydro-5,5,8,8-tétraméthylnaphtalene et 40 ml de DMF. On agite par petites quantités 890 mg (29,6 mmoles) d'hydrure de sodium (80% dans l'huile) et agite jusqu'à cessation du dégagement gazeux. On ajoute ensuite 1,7 ml (27 mmoles) d'iodomethane et agite à température ambiante pendant une heure. On verse le milieu réactionnel dans l'eau, extrait avec de l'éther éthylique, décante la phase organique, sèche sur sulfate de magnésium, évapore. On recueille 7,3 g (99%) du produit attendu sous forme d'une huile qui cristallise lentement. Point de fusion 77-8°C.
(b) acide 2-methoxy-5,6,7,8-tétrahydro-5,5,8,8-tétraméthylnaphtalylboronique.
   De manière analogue à l'exemple 3(a) à partir de 6,7 g (22,5 mmoles) de 3-bromo-2-methoxy-5,6,7,8-tétrahydro-5,5,8,8-tétraméthylnaphtalene, on obtient 4,54 g (77%) d'acide boronique attendu de point de fusion 151-2°C.
(c) acide 3-(3-methoxy-5,6,7,8-tétrahydro-5,5,8,8-tétraméthyl-2-naphtyl)phenyl acrylique.
   De manière analogue à l'exemple 9(b) par réaction de 2,62 g (10 mmoles) d'acide 2-methoxy-5,6,7,8-tétrahydro-5,5,8,8-tétraméthylnaphtylboronique avec 1,51 g (6,7 mmoles) d'acide 3-bromophenylacrylique, on obtient 1,1 g (45%) d'acide 3-(3-methoxy-5,6,7,8-tétrahydro-5,5,8,8-tétraméthyl-2-naphtyl)phenylacrylique de point de fusion 187-8°C.

### EXEMPLE 22

### Acide 3-(3-propyloxy-5,6,7,8-tétrahydro-5,5,8,8-tétraméthyl-2-naphtyl) phenylacrylique.

(a) 3-bromo-2-propyloxy-5,6,7,8-tétrahydro-5,5,8,8-tétraméthylnaphtalene.
   De manière analogue à l'exemple 21(a) par réaction de 7 g (24,7 mmoles) de 3-bromo-2-hydroxy-5,6,7,8-tétrahydro-5,5,8,8-tétraméthylnaphtalene avec 2,45 ml (27 mmoles) de 1-bromopropane, on obtient 8,1 g (100%) de 3-bromo-2-propyloxy-5,6,7,8-tétrahydro-5,5,8,8-tétraméthylnaphtalene sous forme d'une huile orangée.
(b) acide 2-propyloxy-5,6,7,8-tétrahydro-5,5,8,8-tétraméthylnaphtylboronique.
   De manière analogue à l'exemple 3(a) par réaction de 8 g (24,6 mmoles) de 3-bromo-2-propyloxy-5,6,7,8-tétrahydro-5,5,8,8-tétraméthylnaphtalene, on obtient 5,7 g (80%) d'acide boronique attendu de point de fusion 138-9°C.
(c) acide 3-(3-propyloxy-5,6,7,8-tétrahydro-5,5,8,8-tétraméthyl-2-naphtyl)phenyl acrylique.
   De manière analogue à l'exemple 9(b) par réaction de 5 g (17,2 mmoles) d'acide 2-propyloxy-5,6,7,8-tétrahydro-5,5,8,8-tétraméthylnaphtylboronique avec 2,6 g (11,5 mmoles) d'acide 3-bromophenylacrylique, on obtient 1,66 g (35%) d'acide 3-(3-propyloxy-5,6,7,8-tétrahydro-5,5,8,8-tétraméthyl-2-naphtyl)phenylacrylique de point de fusion 172-3°C.

### EXEMPLE 23

### Acide 3-(3-heptyloxy-5,6,7,8-tétrahydro-5,5,8,8-tétraméthyl-2-naphtyl) phenylacrylique.

(a) 3-bromo-2-heptyloxy-5,6,7,8-tétrahydro-5,5,8,8-tétraméthylnaphtalene.
   De manière analogue à l'exemple 21(a) par réaction de 7 g (24,7 mmoles) de 3-bromo-2-hydrpxy-5,6,7,8-tétrahydro-5,5,8,8-tétraméthylnaphtalene avec 4,24 ml (27 mmoles) de 1-bromoheptane, on obtient 10 g (100%) de 3-bromo-2-heptyloxy-5,6,7,8-tétrahydro-5,5,8,8-tétraméthylnaphtalene sous forme d'une huile marron.
(b) acide 2-heptyloxy-5,6,7,8-tétrahydro-5,5,8,8-tétraméthylnaphtylboronique.
   De manière analogue à l'exemple 3(a) à partir de 10 g (26,2 mmoles) de 3-bromo-2-heptyloxy-5,6,7,8-tétrahydro-5,5,8,8-tétraméthylnaphtalene, on obtient 6,1 g (67%) d'acide boronique attendu de point de fusion 102-3°C.
(c) acide 3-(3-heptyloxy-5,6,7,8-tétrahydro-5,5,8,8-tétraméthyl-2-naphtyl)phenyl acrylique.
   De manière analogue à l'exemple 9(b) par réaction de 5 g (14,4 mmoles) d'acide 2-heptyloxy-5,6,7,8-tétrahydro-5,5,8,8-tétraméthylnaphtylboronique avec 2,52 g (11,1 mmoles) d'acide 3-bromophenylacrylique, on obtient 2,7 g (54%) d'acide 3-(3-heptyloxy-5,6,7,8-tétrahydro-5,5,8,8-tétraméthyl-2-naphtyl)phenyl acrylique de point de fusion 112-3°C

### EXEMPLE 24

### Acide 3-(3-methoxymethoxy-5,6,7,8-tétrahydro-5,5,8,8-tétraméthyl-2-naphtyl)phenylacrylique.

(a) 3-bromo-2-methoxymethoxy-5,6,7,8-tétrahydro-5,5,8,8-tétraméthylnaphtalene.
   De manière analogue à l'exemple 21(a) par réaction de 7 g (24,7 mmoles) de 3-bromo-2-hydroxy-5,6,7,8-tétrahydro-5,5,8,8-tétraméthylnaphtalene avec 2,05 ml (27 mmoles) de chlorure de méthoxymethyle, on obtient 8,1 g (100%) de 3-bromo-2-methoxymethoxy-5,6,7,8-tétrahydro-5,5,8,8-tétraméthylnaphtalene sous forme d'une huile marron claire.
(b) acide 2-methoxymethoxy-5,6,7,8-tétrahydro-5,5,8,8-tétraméthylnaphtylboronique.
   De manière analogue à l'exemple 3(a) à partir de 8 g (24,4 mmoles) de 3-bromo-2-methoxymethoxy-5,6,7,8-tétrahydro-5,5,8,8-tétraméthylnaphtalene, on obtient 5,5 g (77%) d'acide boronique attendu de point de fusion 133-4°C.
(c) acide 3-(3-methoxymethoxy-5,6,7,8-tétrahydro-5,5,8,8-tétraméthyl-2-naphtyl) phenylacrylique.
   De maniére analogue à l'exemple 9(b) par réaction de 5,3 g (18,1 mmoles) d'acide 2-methoxymethoxy-5,6,7,8-tétrahydro-5,5,8,8-tétraméthylnaphtylboronique avec 3,16 g (14 mmoles) d'acide 3-bromophenylacrylique, on obtient 4,39 g (80%) d'acide 3-(3-methoxymethoxy-5,6,7,8-tétrahydro-5,5,8,8-tétraméthyl-2-naphtyl) phenylacrylique de point de fusion 156-7°C.

### EXEMPLE 25

### Acide 3-(3-hydroxy-5,6,7,8-tétrahydro-5,5,8,8-tétraméthyl-2-naphtyl)phenylacrylique.

(a) 3-(3-hydroxy-5,6,7,8-tétrahydro-5,5,8,8-tétraméthyl-2-naphtyl) phenylacrylate de methyle.
   Dans un ballon, on introduit 2 g (5 mmoles) d'acide 3-(3-methoxymethoxy-5,6,7,8-tétrahydro-5,5,8,8-tétraméthyl-2-naphtyl)phenylacrylique 10 ml de méthanol et 10 ml de THF. On ajoute 2,8 ml d'acide sulfurique concentré et agite à température ambiante pendant &é heures. On verse le milieu réactionnel dans l'eau, extrait avec de l'éther éthylique, décante la phase organique, lave à l'eau, sèche sur sulfate de magnésium, évapore. On recueille 1,80 g (98%) de l'ester méthylique attendu de point de fusion 182-3°C.
(b) acide 3-(3-hydroxy-5,6,7,8-tétrahydro-5,5,8,8-tétraméthyl-2-naphtyl)phenyl acrylique.
   De manière analogue à l'exemple 1(e) à partir de 1,5 g (4,1 mmoles) de l'ester methylique précédent, on obtient 1,3 g (90%) d'acide 3-(3-hydroxy-5,6,7,8-tétrahydro-5,5,8,8-tétraméthyl-2-naphtyl)phenyl acrylique de point de fusion 244-5°C.

### EXEMPLE 26

### Acide 3-(4,4,7-trimethylthiochromane-6-yl)phenylacrylique.

(a) 1-methyl-3-(3-methylbut-2-enyl)sulfanylbenzene.
   Dans un tricol, on introduit 5 g (40 mmoles) de 3-methylthiophenol 5,6 g (40 mmoles) de carbonate de potassium et 50 ml de DMF. On ajoute 7,2 g (48 mmoles) de bromure de 3-méthyl-2-butene et agite à température ambiante pendant quatre heures. On verse le milieu réactionnel dans l'eau, extrait avec de l'éther éthylique, décante la phase organique, sèche sur sulfate de magnésium, évapore. On recueille 7,8 g (100%) du produit attendu sous forme d'une huile jaune.
(b) 4,4,7-trimethylthiochromane.
   Dans un ballon, on introduit 7 g (36,4 mmoles) de 1-methyl-3-(3-methylbut-2-enyl)sulfanylbenzene 50 ml de toluène et ajoute 10,4 g (54,6 mmoles) d'acide paratoluènesulfonique. On chauffe à reflux pendant quatre heures. On évapore le milieu réactionnel à sec, reprend par l'eau et l'éther éthylique, décante la phase organique, sèche sur sulfate de magnésium, évapore. On recueille 6,8 g (97%) de thiochromane sous forme d'une huile marron.
(c) 6-bromo-4,4,7-trimethylthiochromane.
   Dans un tricol on introduit 6,2 g (32,2 mmoles) de 4,4,7-trimethylthiochromane 40 ml de dichloromethane et 90 mg de poudre de fer. On ajoute 1,65 ml (32,2 mmoles) de brome et agite à température ambiante pendant deux heures. On verse le milieu réactionnel dans une solution de bicarbonate de sodium, extrait avec du dichloromethane, décante la phase organique, sèche sur sulfate de magnésium, évapore. Le résidu obtenu est purifié par chromatographie sur colonne de silice, élué avec de l'heptane. On recueille 5,9 g (67%) de dérivé bromé sous forme d'une huile jaune claire.
(d) acide 4,4,7-trimethylthiochromanylboronique.
   De manière analogue à l'exemple 3(a) à partir de 5,8 g (21,4 mmoles) de 6-bromo-4,4,7-trimethylthiochromane, on obtient 3,88 g (76%) d'acide boronique attendu de point de fusion 252-3°C.
(e) acide 3-(4,4,7-trimethylthiochromane-6-yl)phenylacrylique.
   De manière analogue à l'exemple 9(b) par réaction de 1,5 g (6,3 mmoles) d'acide 4,4,7-trimethylthiochromanylboronique avec 1,2 g (5,3 mmoles) d'acide 3-bromophenylacrylique, on obtient 1,1 g (99%) d'acide 3-(4,4,7-trimethylthio chromane-6-yl)phenylacrylique de point de fusion 102-3°C

### EXEMPLE 27

### N-ethyl-3-(3,5,5,8,8-pentaméthyl-5,6,7,8-tétrahydro-2-naphtyl)phenyl acrylamide.

(a) chlorure de 3-(3,5,5,8,8-pentaméthyl-5,6,7,8-tétrahydro-2-naphtyl)phenyl acryloyle.
   Dans un ballon, on introduit 3,5 g (10 mmoles) d'acide 3-(3,5,5,8,8-pentaméthyl-5,6,7,8-tétrahydro-2-naphtyl)phenylacrylique 50 ml de dichloromethane et ajoute 2 ml (10 mmoles) de dicyclohexylamine. On agite à température ambiante 10 minutes puis introduit 729 µl (10 mmoles) de chlorure de thionyle et agite 15 minutes. On évapore à sec le milieu réactionnel, reprend par l'éther éthylique, filtre le sel de dicyclohexylamine, évapore le filtrat. On recueille 3,7 g (100%) de chlorure d'acide brut qui sera utilisé telquel pour la suite de la synthèse.
(b) N-ethyl-3-(3,5,5,8,8-pentaméthyl-5,6,7,8-tétrahydro-2-naphtyl)phenylacrylamide.
   Dans un ballon, on introduit 20 ml de THF et ajoute 2,8 ml (35 mmoles) d'une solution d'éthylamine (70%). On ajoute goutte à goutte une solution de 1,2 g (3,2 mmoles) de chlorure de 3-(3,5,5,8,8-pentaméthyl-5,6,7,8-tétrahydro-2-naphtyl) phenyl acryloyle dans 40 ml de THF et agite à température ambiante une heure. On acidifie le milieu réactionnel avec de l'acide chlorhydrique, extrait avec de l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 817 mg (68%) de N-ethyl-3-(3,5,5,8,8-pentaméthyl-5,6,7,8-tétrahydro-2-naphtyl)phenylacrylamide de point de fusion 158-9°C.

### EXEMPLE 28

### N-(4-hydroxyphenyl)-3-(3,5,5,8,8-pentaméthyl-5,6,7,8-tétrahydro-2-naphtyl) phenylacrylamide.

De manière analogue à l'exemple 27(a) par réaction de 1,2 g (3,2 mmoles) de chlorure de 3-(3,5,5,8,8-pentaméthyl-5,6,7,8-tétrahydro-2-naphtyl)phenyl acryloyle avec 349 mg (3,2 mmoles) de 4-hydroxyaniline, on obtient 810 mg (57%) de N-(4-hydroxyphenyl)-3-(3,5,5,8,8-pentaméthyl-5,6,7,8-tétrahydro-2-naphtyl)phenyl acrylamide de point de fusion 240-1°C.

### EXEMPLE 29

### Morpholide de l'acide 3-(3,5,5,8,8-pentaméthyl-5,6,7,8-tétrahydro-2-naphtyl) phenylacrylique.

De manière analogue à l'exemple 27(a) par réaction de 1,3 g (3,4 mmoles) de chlorure de 3-(3,5,5,8,8-pentaméthyl-5,6,7,8-tétrahydro-2-naphtyl)phenyl acryloyle avec 620 µl (7,12 mmoles) de morpholine, on obtient 1,25 g (88%) de morpholide de l'acide 3-(3,5,5,8,8-pentaméthyl-5,6,7,8-tétrahydro-2-naphtyl)phenylacrylique de point de fusion 158-9°C.

### EXEMPLE 30

### 3-(3,5,5,8,8-pentaméthyl-5,6,7,8-tétrahydro-2-naphtyl)phenylacrylate d'éthyle.

De manière analogue à l'exemple 1(d) par réaction de 5 g (16,3 mmoles) de 3-(3,5,5,8,8-pentaméthyl-5,6,7,8-tétrahydro-2-naphtyl)benzaldehyde avec 4,79 g (21,2 mmoles) de triéthylphosphonoacétate, on obtient 5 g (81%) de 3-(3,5,5,8,8-pentaméthyl-5,6,7,8-tétrahydro-2-naphtyl)phenylacrylate d'éthyle de point de fusion 70-2°C.

### EXEMPLE 31

### Acide 3-[3-(3,5,5,8,8-pentaméthyl-5,6,7,8-tétrahydro-2-naphtyl)phenyl]but-2-enoique.

(a) 3-(3,5,5,8,8-pentaméthyl-5,6,7,8-tétrahydro-2-naphtyl)acetophenone.
   De manière analogue à l'exemple 9(b) par réaction de 5 g (20,3 mmoles) d'acide 3,5,5,8,8-pentaméthyl-5,6,7,8-tétrahydro-2-naphtylboronique avec 2,7 g (13,5 mmoles) de 3-bromoacetophenone, on obtient 4,3 g (90%) de produit attendu de point de fusion 89-90°C.
(b) 3-[3-(3,5,5,8,8-pentaméthyl-5,6,7,8-tétrahydro-2-naphtyl)phenyl]but-2-enoate d'éthyle.
   De manière analogue à l'exemple 1(d) par réaction de 3,7 g (11,5 mmoles) de 3-(3,5,5,8,8-pentaméthyl-5,6,7,8-tétrahydro-2-naphtyl)acetophenone avec 3,9 g (17,3 mmoles) de triéthylphosphonoacétate, on obtient 2,67 g (60%) de l'ester éthylique attendu sous forme d'une huile jaune.
(c) acide 3-[3-(3,5,5,8,8-pentaméthyl-5,6,7,8-tétrahydro-2-naphtyl)phenyl]but-2-enoique.
   De manière analogue à l'exemple 1(e) à partir de 2,5 g (6,4 mmoles) de l'ester éthylique précédent, on obtient 1,63 g (70%) d'acide 3-[3-(3,5,5,8,8-pentaméthyl-5,6,7,8-tétrahydro-2-naphtyl)phenyl]but-2-enoique de point de fusion 166-7°C.

### EXEMPLE 32

Dans cet exemple, on a illustré diverses formulations concrètes à base des composés selon l'invention.

### A- VOIE ORALE

(a) Comprimé de 0,2 g
   - Composé de l'exemple 1 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 3 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé de l'exemple 5 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 2 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 21 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 9 0,300 g
   - Vaseline blanche codex 100 g
(c) Crème Eau-dans-Huile non ionique
   - Composé de l'exemple 7 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 30 0,100 g
   - Polyéthylène glycol (PEG 400) 69,900 g
   - Ethanol à 95% 30,000 g
(e) Onguent hydrophobe
   - Composé de l'exemple 25 0,300 g
   - Mirystate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) 100g
(f) Crème Huile-dans-Eau non ionique
   - Composé de l'exemple 14 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile 100 g

## Revendications

1. Composés bicycliques-aromatiques, **caractérisés par le fait qu'**ils répondent à la formule générale (I) suivante : 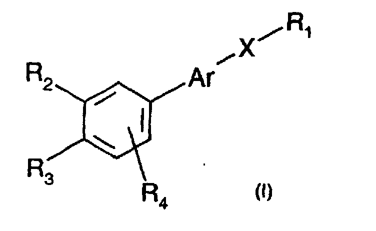 dans laquelle:
- R₁ représente
(i) le radical -CH₃
(ii) le radical -CH₂OR₅
(iii) le radical -CO-R₆
R₅ et R₆ ayant la signification donnée ci-après
- Ar représente un radical choisi parmi les radicaux de formules (a)-(e) suivantes: 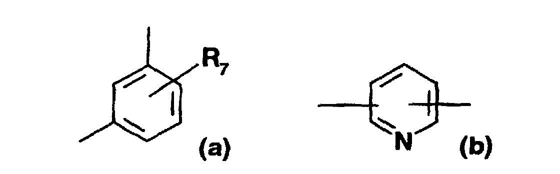 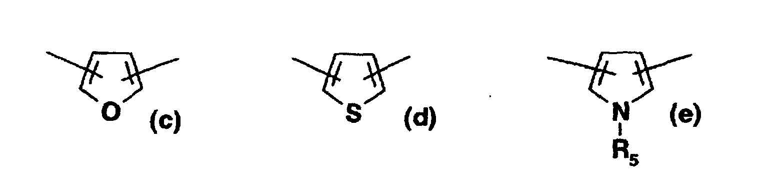 R₅ et R₇ ayant la signification donnée ci-après,
- X représente 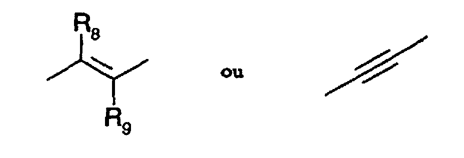 R₈ et R₉ ayant les significations données ci-après
- R₂ et R₃, identiques ou différents, représentent
(i) un atome d'hydrogène,
(ii) un radical alkyle présentant au moins 3 atomes de carbone, parmi lesquels le carbone attaché au radical phényl est au moins substitué par deux atomes de carbone,
(iii) un radical -OR₅,
(iv) un radical -SR₅,
R₅ ayant la signification donnée ci-après,
étant entendu que R₂ et R₃ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
et étant entendu que R₂ et R₃ ne peuvent pas avoir en même temps les significations (i), (iii) et (iv) mentionnées ci-dessus.
- R₄ et R₇, identiques ou différents, représentent un atome d'hydrogène, un atome d'halogène, un radical alkyle linéaire ou ramifié ayant de 1 à 20 atomes de carbone, un radical -OR₅,
- R₅ représente un atome d'hydrogène, un radical alkyle ayant de 1 à 12 atomes de carbone ou un radical -COR₁₀
R₁₀ ayant la signification donnée ci-après,
- R₆ représente:
(a) un atome d'hydrogène
(b) un radical alkyle ayant de 1 à 12 atomes de carbone
(c) un radical de formule: 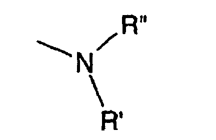 R' et R" ayant la signification donnée ci-après,
(d) un radical -OR₁₁
R₁₁ ayant la signification donnée ci-après,
- R₈ et R₉, identiques ou différents, représentent un atome d'hydrogène ou un radical alkyle ayant de 1 à 12 atomes de carbone,
- R₁₀ représente un radical alkyle ayant de 1 à 12 atomes de carbone,
- R₁₁ représente un atome d'hydrogène, un radical alkyle linéaire ou ramifié, ayant de 1 à 20 atomes de carbone, un radical alkényl, un radical mono ou polyhydroxyalkyle, un radical aryle ou aralkyle, éventuellement substitué par au moins un atome d'halogène, un hydroxyle ou une fonction nitro , ou un reste de sucre ou un reste d'amino acide ou de peptide,
- R' et R", identiques ou différents, représentent un atome d'hydrogène, un radical alkyle ayant 1 à 12 atomes de carbone, un radical mono ou polyhydroxyalkyle, un radical aryle éventuellement substitué par au moins un atome d'halogène, un hydroxyle ou une fonction nitro, ou un reste d'amino acide ou de sucre ou encore pris ensemble forment un hétérocycle,
ainsi que leurs sels et leurs isomères optiques et géométriques.

2. Composés selon la revendication 1, **caractérisés par le fait qu'**ils se présentent sous forme de sels d'un métal alcalin ou alcalino-terreux, ou encore de zinc ou d'une amine organique.

3. Composés selon la revendication 1, **caractérisés par le fait qu'**ils sont pris, seuls ou en mélanges, dans le groupe constitué par :
- Acide 5-(3-tert-butyl-4-méthoxyphényl)-2-thiophèneacrylique,
- Acide 5-(3-tert-butyl-4-méthoxyphényl)-2-thiophènepropiolique,
- Acide 2-(3-tert-butyl-4-méthoxyphényl)-4-thiophèneacrylique,
- Acide 4-(3-tert-butyl-4-méthoxyphényl)-2-thiophèneacrylique,
- Acide 5-(3,5,5,8,8-pentaméthyl-5,6,7,8-tétrahydro-2-naphtyl)-2-thiophène acrylique,
- Acide 4-(3,5,5,8,8-pentaméthyl-5,6,7,8-tétrahydro-2-naphtyl)-2-thiophène acrylique,
- Acide 4-(5,6,7,8-tétrahydro-5,5,8,8-tétraméthyl-2-naphtyl)-2-thiophene acrylique,
- Acide 5-(3,5,5,8,8-pentaméthyl-5,6,7,8-tétrahydro-2-naphtyl)-2-thiophène propiolique,
- Acide 3-(3,5,5,8,8-pentaméthyl-5,6,7,8-tétrahydro-2-naphtyl)phenyl propiolique,
- Acide N-méthyl-4-(5,6,7,8-tétrahydro-5,5,8,8-tétraméthyl-2-naphtyl)-2-pyrroleacrylique,
- Acide 3-(5,6,7,8-tétrahydro-5,5,8,8-tétraméthyl-2-naphtyl)phenylacrylique,
- Acide N-méthyl-4-(3,5,5,8,8-pentaméthyl-5,6,7,8-tétrahydro-2-naphtyl)-2-pyrroleacrylique,
- Acide 4-(3,5,5,8,8-pentaméthyl-5,6,7,8-tétrahydro-2-naphtyl)-2-pyrrole acrylique,
- Acide 3-(3,5,5,8,8-pentaméthyl-5,6,7,8-tétrahydro-2-naphtyl)phenylacrylique,
- Acide 3-(5,6,7,8-tétrahydro-5,5,8,8-tétraméthyl2-naphtyl)phenylpropiolique,
- Acide 2-methoxy-3-(3,5,5,8,8-pentaméthyl-5,6,7,8-tétrahydro-2-naphtyl) phenylacrylique,
- Acide 2-propyloxy-3-(3,5,5,8,8-pentaméthyl-5,6,7,8-tétrahydro-2-naphtyl) phenylacrylique,
- Acide 2-heptyloxy-3-(3,5,5,8,8-pentaméthyl-5,6,7,8-tétrahydro-2-naphtyl) phenylacrylique,
- Acide 2-methoxymethoxy-3-(3,5,5,8,8-pentaméthyl-5,6,7,8-tétrahydro-2-naphtyl) phenylacrylique,
- Acide 2-hydroy-3-(3,5,5,8,8-pentaméthyl-5,6,7,8-tétrahydro-2-naphtyl) phenylacrylique,
- Acide 3-(3-methoxy-5,6,7,8-tétrahydro-5,5,8,8-tétraméthyl-2-naphtyl) phenylacrylique,
- Acide 3-(3-propyloxy-5,6,7,8-tétrahydro-5,5,8,8-tétraméthyl-2-naphtyl) phenylacrylique,
- Acide 3-(3-heptyloxy-5,6,7,8-tétrahydro-5,5,8,8-tétraméthyl-2-naphtyl) phenylacrylique,
- Acide 3-(3-methoxymethoxy-5,6,7,8-tétrahydro-5,5,8,8-tétraméthyl-2-naphtyl)phenylacrylique,
- Acide 3-(3-hydroxy-5,6,7,8-tétrahydro-5,5,8,8-tétraméthyl-2-naphtyl) phenylacrylique,
- Acide 3-(4,4,7-trimethylthiochromane-6-yl)phenylacrylique,
- N-ethyl-3-(3,5,5,8,8-pentaméthyl-5,6,7,8-tétrahydro-2-naphtyl)phenyl acrylamide,
- N-(4-hydroxyphenyl)-3-(3,5,5,8,8-pentaméthyl-5,6,7,8-tétrahydro-2-naphtyl) phenylacrylamide,
- Morpholide de l'acide 3-(3,5,5,8,8-pentaméthyl-5,6,7,8-tétrahydro-2-naphtyl)phenylacrylique,
- 3-(3,5,5,8,8-pentaméthyl-5,6,7,8-tétrahydro-2-naphtyl)phenylacrylate d'éthyle,
- Acide 3-[3-(3,5,5,8,8-pentaméthyl-5,6,7,8-tétrahydro-2-naphtyl)phenyl]but-2-enoique,
- Acide 4-methoxymethoxy-3-(3,5,5,8,8-pentaméthyl-5,6,7,8-tétrahydro-2-naphtyl)phenylacrylique,
- Acide 4-hydroxy-3-(3,5,5,8,8-pentaméthyl-5,6,7,8-tétrahydro-2-naphtyl) phenylacrylique,
- Acide 4-propyloxy-3-(3,5,5,8,8-pentaméthyl-5,6,7,8-tétrahydro-2-naphtyl) phenylacrylique,
- Acide 4-heptyloxy-3-(3,5,5,8,8-pentaméthyl-5,6,7,8-tétrahydro-2-naphtyl) phenylacrylique,
- 3-(3,5,5,8,8-pentaméthyl-5,6,7,8-tétrahydro-2-naphtyl)phenyl]acroleine,
- 3-[3-(3,5,5,8,8-pentaméthyl-5,6,7,8-tétrahydro-2-naphtyl)phenyl]prop-2-en-1-ol,
- Acide *cis*-3-[3-(3,5,5,8,8-pentaméthyl-5,6,7,8-tétrahydro-2-naphtyl)phenyl] but-2-enoique,
- Acide *cis*-3-(3,5,5,8,8-pentaméthyl-5,6,7,8-tétrahydro-2-naphtyl)phenyl acrylique,
- Acide 5-(3,5,5,8,8-pentaméthyl-5,6,7,8-tétrahydro-2-naphtyl)-3-pyridine acrylique,
- Acide -3-(3-butyl-5,6,7,8-tétrahydro-5,5,8,8-tétraméthyl-2-naphtyl)phenyl acrylique,
- Acide 6-(3,5,5,8,8-pentaméthyl-5,6,7,8-tétrahydro-2-naphtyl)-2-pyridine acrylique.

4. Composés selon la revendication 1, **caractérisés par le fait qu'**ils présentent au moins l'une des, et de préférence toutes les, caractéristiques suivantes :
- R₁ représente le radical -CO-R₆
- Ar représente les radicaux de formule (a) ou (d)
- X représente le radical 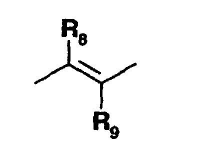
- R₂ et R₃ pris ensemble forment avec le cycle aromatique adjacent un cycle à 5 ou 6 chainons éventuellement substitué par des groupes méthyle et/ou éventuellement interrompu par un atome d'oxygène ou de soufre.

5. Composés selon la revendication 1, **caractérisés par le fait que** le composé est l'acide 4-hydroxy-3-(3,5,5,8,8-pentaméthyl-5,6,7,8-tétrahydro-2-naphtyl) phenylacrylique.

6. Composé selon la revendication 5 à titre de médicament.

7. Utilisation du composé selon la revendication 6 pour la fabrication d'un médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les coméopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particuliérement les leucémies promyélocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose ou l'hypertension ainsi que le diabète non-insulino dépendant, pour le traitement de désordres cutanés dus à une exposition aux rayonnements U.V..

8. Composés selon l'une quelconque des revendications 1 à 4 à titre de médicament.

9. Utilisation d'un composé selon la revendication 8 pour la fabrication d'un médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les cornéopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particuliérement les leucémies promyélocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose ou l'hypertension ainsi que le diabète non-insulino dépendant, pour le traitement de désordres cutanés dus à une exposition aux rayonnements U.V..

10. Composition pharmaceutique, **caractérisée par le fait qu'**elle comprend, dans un support pharmaceutiquement acceptable, au moins l'un des composés tels que définis à l'une quelconque des revendications 1 à 5.

11. Composition selon la revendication 10, **caractérisée en ce que** la concentration en composé(s) selon l'une des revendication 1 à 5 est comprise entre 0,001 % et 5 % en poids par rapport à l'ensemble de la composition.

12. Composition cosmétique, **caractérisée par le fait qu'**elle comprend, dans un support cosmétiquement acceptable, au moins l'un des composés tels que définis à l'une quelconque des revendications 1 à 5.

13. Composition selon la revendication 12, **caractérisée en ce que** la concentration en composé(s) selon l'une des revendications 1 à 5 est comprise entre 0,001 % et 3 % en poids par rapport à l'ensemble de la composition.

14. Utilisation d'une composition selon les revendications 12 ou 13 pour la preparation d'une composition pour l'hygiène corporelle ou capillaire.

## Claims

1. Bicyclic aromatic compounds, **characterized in that** they correspond to the general formula (I) below: 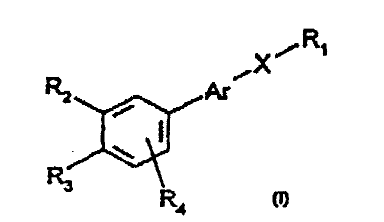 in which:
- R₁ represents
(i) the -CH₃ radical
(ii) the radical -CH₂OR₅
(iii) the radical -COR₆
R₅ and R₆ having the meaning given below
- Ar represents a radical chosen from the radicals of formulae (a)-(e) below: 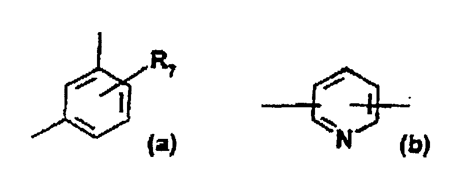 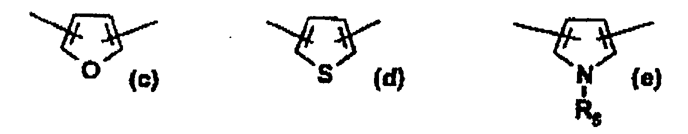 R₅ and R₇ having the meaning given below,
- X represents 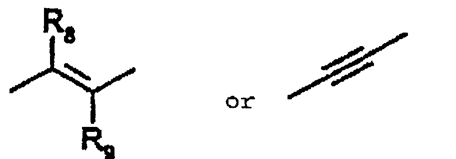 R₈ and R₉ having the meanings given below
- R₂ and R₃, which may be identical or different, represent
(i) a hydrogen atom,
(ii) an alkyl radical having at least 3 carbon atoms, among which the carbon attached to the phenyl radical is substituted with at least two carbon atoms,
(iii) a radical -OR₅,
(iv) a radical -SR₅,
R₅ having the meaning given below,
it being understood that R₂ and R₃, taken together, may form with the adjacent aromatic ring a 5- or 6-membered ring optionally substituted with methyl groups and/or optionally interrupted by an oxygen or sulphur atom,
and it being understood that R₂ and R₃ cannot at the same time have the meanings (i), (iii) and (iv) mentioned above,
- R₄ and R₇, which may be identical or different, represent a hydrogen atom, a halogen atom, a linear or branched alkyl radical having from 1 to 20 carbon atoms or a radical -OR₅,
- R₅ represents a hydrogen atom, an alkyl radical having from 1 to 12 carbon atoms or a radical -COR₁₀
R₁₀ having the meaning given below,
- R₆ represents:
(a) a hydrogen atom
(b) an alkyl radical having from 1 to 12 carbon atoms
(c) a radical of formula: 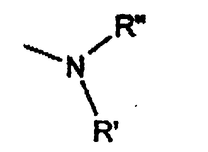 R' and R" having the meaning given below,
(d) a radical -OR₁₁
R₁₁ having the meaning given below,
- R₈ and R₉, which may be identical or different, represent a hydrogen atom or an alkyl radical having from 1 to 12 carbon atoms,
- R₁₀ represents an alkyl radical having from 1 to 12 carbon atoms,
- R₁₁ represents a hydrogen atom, a linear or branched alkyl radical having from 1 to 20 carbon atoms, an alkenyl radical, a mono- or polyhydroxyalkyl radical, an aryl or aralkyl radical, optionally substituted with at least one halogen atom, a hydroxyl or a nitro functional group, a sugar residue or an amino acid or peptide residue,
- R' and R", which may be identical or different, represent a hydrogen atom, an alkyl radical having from 1 to 12 carbon atoms, a mono- or polyhydroxyalkyl radical, an aryl radical, optionally substituted with at least one halogen atom, a hydroxyl or a nitro functional group, or an amino acid or sugar residue, or alternatively, taken together form a heterocycle, as well as the salts thereof and the optical and geometrical isomers thereof.

2. Compounds according to Claim 1, **characterized in that** they are in the form of salts of an alkali metal or alkaline-earth metal, or alternatively of zinc or of an organic amine.

3. Compounds according to Claim 1, **characterized in that** they are taken, alone or as mixtures, from the group consisting of:
- 5-(3-tert-Butyl-4-methoxyphenyl)-2-thiopheneacrylic acid,
- 5-(3-tert-Butyl-4-methoxyphenyl)-2-thiophenepropiolic acid,
- 2-(3-tert-Butyl-4-methoxyphenyl)-4-thiopheneacrylic acid,
- 4-(3-tert-Butyl-4-methoxyphenyl)-2-thiopheneacrylic acid,
- 5-(3,5,5,8,8-Pentamethyl-5,6,7,8-tetrahydro-2-naphthyl)-2-thiopheneacrylic acid,
- 4-(3,5,5,8,8-Pentamethyl-5,6,7,8-tetrahydro-2-naphthyl)-2-thiopheneacrylic acid,
- 4-(5,6,7,8-Tetrahydro-5,5,8,8-tetramethyl-2-naphthyl)-2-thiopheneacrylic acid,
- 5-(3,5,5,8,8-Pentamethyl-5,6,7,8-tetrahydro-2-naphthyl)-2-thiophenepropiolic acid,
- 3-(3,5,5,8,8-Pentamethyl-5,6,7,8-tetrahydro-2-naphthyl)phenylpropiolic acid,
- N-Methyl-4-(5,6,7,8-tetrahydro-5,5,8,8-tetramethyl-2-naphthyl)-2-pyrroleacrylic acid,
- 3-(5,6,7,8-Tetrahydro-5,5,8,8-tetramethyl-2-naphthyl)phenylacrylic acid,
- N-Methyl-4-(3,5,5,8,8-pentamethyl-5,6,7,8-tetrahydro-2-naphthyl)-2-pyrroleacrylic acid,
- 4-(3,5,5,8,8-Pentamethyl-5,6,7,8-tetrahydro-2-naphthyl)-2-pyrroleacrylic acid,
- 3-(3,5,5,8,8-Pentamethyl-5,6,7,8-tetrahydro-2-naphthyl)phenylacrylic acid,
- 3-(5,6,7,8-Tetrahydro-5,5,8,8-tetramethyl-2-naphthyl)phenylpropiolic acid,
- 2-Methoxy-3-(3,5,5,8,8-pentamethyl-5,6,7,8-tetrahydro-2-naphthyl)phenylacrylic acid,
- 2-Propyloxy-3-(3,5,5,8,8-pentamethyl-5,6,7,8-tetrahydro-2-naphthyl)phenylacrylic acid,
- 2-Heptyloxy-3-(3,5,5,8,8-pentamethyl-5,6,7,8-tetrahydro-2-naphthyl)phenylacrylic acid,
- 2-Methoxymethoxy-3-(3,5,5,8,8-pentamethyl-5,6,7,8-tetrahydro-2-naphthyl)phenylacrylic acid,
- 2-Hydroxy-3-(3,5,5,8,8-pentamethyl-5,6,7,8-tetrahydro-2-naphthyl)phenylacrylic acid,
- 3-(3-Methoxy-5,6,7,8-tetrahydro-5,5,8,8-tetramethyl-2-naphthyl)phenylacrylic acid,
- 3-(3-Propyloxy-5,6,7,8-tetrahydro-5,5,8,8-tetramethyl-2-naphthyl)phenylacrylic acid,
- 3-(3-Heptyloxy-5,6,7,8-tetrahydro-5,5,8,8-tetramethyl-2-naphthyl)phenylacrylic acid,
- 3-(3-Methoxymethoxy-5,6,7,8-tetrahydro-5,5,8,8-tetramethyl-2-naphthyl)phenylacrylic acid,
- 3-(3-Hydroxy-5,6,7,8-tetrahydro-5,5,8,8-tetramethyl-2-naphthyl)phenylacrylic acid,
- 3-(4,4,7-Trimethylthiochroman-6-yl)-phenylacrylic acid,
- N-Ethyl-3-(3,5,5,8,8-pentamethyl-5,6,7,8-tetrahydro-2-naphthyl)phenylacrylamide,
- N-(4-Hydroxyphenyl)-3-(3,5,5,8,8-pentamethyl-5,6,7,8-tetrahydro-2-naphthyl)phenylacrylamide,
- 3-(3,5,5,8,8-Pentamethyl-5,6,7,8-tetrahydro-2-naphthyl)phenylacrylic acid morpholide,
- Ethyl 3-(3,5,5,8,8-pentamethyl-5,6,7,8-tetrahydro-2-naphthyl)phenylacrylate,
- 3-[3-(3,5,5,8,8-Pentamethyl-5,6,7,8-tetrahydro-2-naphthyl)phenyl]but-2-enoic acid,
- 4-Methoxymethoxy-3-(3,5,5,8,8-pentamethyl-5,6,7,8-tetrahydro-2-naphthyl)phenylacrylic acid,
- 4-Hydroxy-3-(3,5,5,8,8-pentamethyl-5,6,7,8-tetrahydro-2-naphthyl)phenylacrylic acid,
- 4-Propyloxy-3-(3,5,5,8,8-pentamethyl-5,6,7,8-tetrahydro-2-naphthyl)phenylacrylic acid,
- 4-Heptyloxy-3-(3,5,5,8,8-pentamethyl-5,6,7,8-tetrahydro-2-naphthyl)phenylacrylic acid,
- 3-(3,5,5,8,8-Pentamethyl-5,6,7,8-tetrahydro-2-naphthyl)phenyl]acrolein,
- 3-[3-(3,5,5,8,8-pentamethyl-5,6,7,8-tetrahydro-2-naphthyl)phenyl]prop-2-en-1-ol,
- cis-3-[3-(3,5,5,8,8-Pentamethyl-5,6,7,8-tetrahydro-2-naphthyl)phenyl]but-2-enoic acid,
- cis-3-(3,5,5,8,8-Pentamethyl-5,6,7,8-tetrahydro-2-naphthyl)phenylacrylic acid,
- 5-(3,5,5,8,8-Pentamethyl-5,6,7,8-tetrahydro-2-naphthyl)-3-pyridineacrylic acid,
- 3-(3-Butyl-5,6,7,8-tetrahydro-5,5,8,8-tetramethyl-2-naphthyl)phenylacrylic acid,
- 6-(3,5,5,8,8-Pentamethyl-5,6,7,8-tetrahydro-2-naphthyl)-2-pyridineacrylic acid.

4. Compounds according to Claim 1, **characterized in that** they have at least one, and preferably all, of the following characteristics:
- R₁ represents the radical -COR₆
- Ar represents the radicals of formula (a) or (d)
- X represents the radical 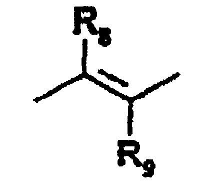
- R₂ and R₃, taken together, form, with the adjacent aromatic ring, a 5- or 6-membered ring optionally substituted with methyl groups and/or optionally interrupted by an oxygen or sulphur atom.

5. Compounds according to Claim 1, **characterized in that** the compound is 4-hydroxy-3-(3,5,5,8,8-pentamethyl-5,6,7,8-tetrahydro-2-napthyl) phenylacrylic acid.

6. Compound according to Claim 5, as a medicinal product.

7. Use of the compound according to Claim 6, for the manufacture of a medicinal product intended for the treatment of dermatological complaints associated with a keratinization disorder which has a bearing on differentiation and on proliferation, in particular for treating common acne, comedones, polymorphonuclear leukocytes, rosacea, nodulocystic acne, acne conglobata, senile acne and secondary acnes such as solar, medication-related or profession-related acne; for treating other types of keratinization disorder, in particular ichthyosis, ichthyosiform states, Darier's disease, palmoplantar keratoderma, leucoplasias and leucoplasiform states, and cutaneous or mucous (buccal) lichen; for treating other dermatological complaints associated with a keratinization disorder with an inflammatory and/or immunoallergic component and, in particular, all forms of psoriasis, whether it is cutaneous, mucous or ungual psoriasis and even psoriatic rheumatism, or alternatively cutaneous atopy, such as eczema or respiratory atopy or alternatively gingival hypertrophy; the compounds may also be used for some inflammatory complaints which show no keratinization disorder; for treating all dermal or epidermal hyperproliferations, whether benign or malignant and whether they are of viral origin or otherwise, such as common warts, flat warts and verruciform epidermodysplasia, oral or florid papillomatoses and hyperproliferations which may be induced by ultraviolet radiation, in particular in the case of basocellular and spinocellular epithelioma; for treating other dermatological disorders such as bullosis and collagen diseases; for treating certain ophthalmological disorders, in particular corneopathies; for repairing or combating ageing of the skin, whether this is light-induced or chronological ageing, or for reducing actinic keratoses and pigmentations, or any pathologies associated with chronological or actinic ageing; for preventing or curing the stigmata of epidermal and/or dermal atrophy induced by local or systemic corticosteroids, or any other form of cutaneous atrophy; for preventing or treating cicatrization disorders or for preventing or repairing stretchmarks; for favouring cicatrization, for combating disorders of sebaceous functioning such as the hyperseborrhoea of acne or simple seborrhoea; in the treatment or prevention of cancerous or precancerous states, more particularly promyelocytary leukemia; in the treatment of inflammatory complaints such as arthritis; in the treatment of any general or skin complaint of viral origin; in the prevention or treatment of alopecia; in the treatment of dermatological complaints having an immunological component; in the treatment of complaints of the cardiovascular system such as arteriosclerosis or hypertension, as well as insulin-independent diabetes, in the treatment of skin disorders caused by exposure to UV radiation.

8. Compounds according to any one Claims 1 to 4, as a medicinal product.

9. Use of a compound according to Claim 8, for the manufacture of a medicinal product intended for the treatment of dermatological complaints associated with a keratinization disorder which has a bearing on differentiation and on proliferation, in particular for treating common acne, comedones, polymorphonuclear leukocytes, rosacea, nodulocystic acne, acne conglobata, senile acne and secondary acnes such as solar, medication-related or profession-related acne; for treating other types of keratinization disorder, in particular ichthyosis, ichthyosiform states, Darier's disease, palmoplantar keratoderma, leucoplasias and leucoplasiform states, and cutaneous or mucous (buccal) lichen; for treating other dermatological complaints associated with a keratinization disorder with an inflammatory and/or immunoallergic component and, in particular, all forms of psoriasis, whether it is cutaneous, mucous or ungual psoriasis and even psoriatic rheumatism, or alternatively cutaneous atopy, such as eczema or respiratory atopy or alternatively gingival hypertrophy; the compounds may also be used for some inflammatory complaints which show no keratinization disorder; for treating all dermal or epidermal hyperproliferations, whether benign or malignant and whether they are of viral origin or otherwise, such as common warts, flat warts and verruciform epidermodysplasia, oral or florid papillomatoses and hyperproliferations which may be induced by ultraviolet radiation, in particular in the case of basocellular and spinocellular epithelioma; for treating other dermatological disorders such as bullosis and collagen diseases; for treating certain ophthalmological disorders, in particular corneopathies; for repairing or combating ageing of the skin, whether this is light-induced or chronological ageing, or for reducing actinic keratoses and pigmentations, or any pathologies associated with chronological or actinic ageing; for preventing or curing the stigmata of epidermal and/or dermal atrophy induced by local or systemic corticosteroids, or any other form of cutaneous atrophy; for preventing or treating cicatrization disorders or for preventing or repairing stretchmarks; for favouring cicatrization, for combating disorders of sebaceous functioning such as the hyperseborrhoea of acne or simple seborrhoea; in the treatment or prevention of cancerous or precancerous states, more particularly promyelocytary leukemia; in the treatment of inflammatory complaints such as arthritis; in the treatment of any general or skin complaint of viral origin; in the prevention or treatment of alopecia; in the treatment of dermatological complaints having an immunological component; in the treatment of complaints of the cardiovascular system such as arteriosclerosis or hypertension, as well as insulin-independent diabetes, in the treatment of skin disorders caused by exposure to UV radiation.

10. Pharmaceutical composition, **characterized in that** it comprises, in a pharmaceutically acceptable support, at least one of the compounds as defined in any one of Claims 1 to 5.

11. Composition according to Claim 10, **characterized in that** the concentration of compound(s) according to one of Claims 1 to 5 is between 0.001% and 5% by weight relative to the composition as a whole.

12. Cosmetic composition, **characterized in that** it comprises, in a cosmetically acceptable support, at least one of the compounds as defined in any one of Claims 1 to 5.

13. Composition according to Claim 12, **characterized in that** the concentration of compound(s) according to one of Claims 1 to 5 is between 0.001% and 3% by weight relative to the composition as a whole.

14. Use of a composition according to Claims 12 or 13, for the preparation of a composition for body or hair hygiene.

## Patentansprüche

1. Bicyclisch-aromatische Verbindungen, **dadurch gekennzeichnet, daß** sie durch die folgende allgemeine Formel (I) dargestellt werden können: 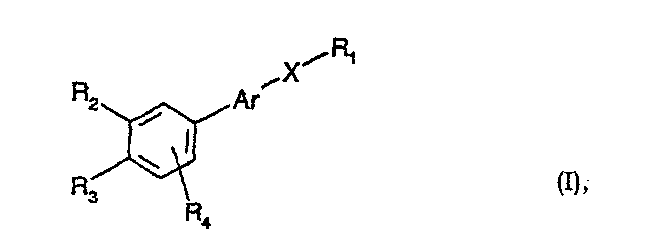 worin bedeuten:
- R₁
(i) die Gruppe -CH₃,
(ii) eine Gruppe -CH₂OR₅,
(iii) eine Gruppe -CO-R₆,
wobei R₅ und R₆ die nachfolgend angegebenen Bedeutungen aufweisen;
- Ar eine Gruppe, die unter den folgenden Gruppen (a) bis (e) ausgewählt ist: 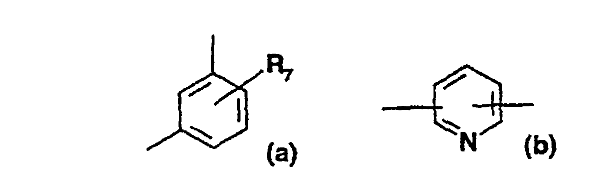 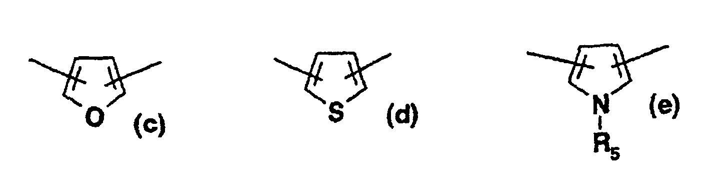 wobei die Gruppen R₅ und R₇ die nachfolgend angegebenen Bedeutungen aufweisen;
- X: 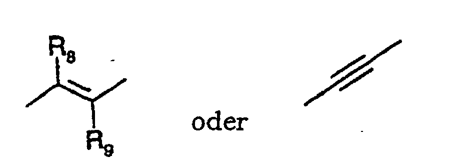 wobei die Gruppen R₈ und R₉ die nachfolgend angegebenen Bedeutungen aufweisen;
- R₂ und R₃, die identisch oder voneinander verschieden sind:
(i) ein Wasserstoffatom,
(ii) eine Alkylgruppe mit mindestens 3 Kohlenstoffatomen, wobei das an den Phenylring gebundene Kohlenstoffatom mit mindestens zwei Kohlenstoffatomen substituiert ist,
(iii) eine Gruppe -OR₅,
(iv) eine Gruppe -SR₅, wobei R₅ die nachfolgend angegebenen Bedeutungen hat;
mit der Maßgabe, daß die Gruppen R₂ und R₃ mit dem angrenzenden aromatischen Ring auch einen 5- oder 6-gliedrigen Ring bilden können, der gegebenenfalls mit Methylgruppen substituiert und/oder gegebenenfalls durch ein Sauerstoff- oder Schwefelatom unterbrochen ist; und
mit der Maßgabe, daß die Gruppen R₂ und R₃ nicht gleichzeitig die oben aufgeführten Bedeutungen (i), (iii) und (iv) haben können;
- R₄ und R₇, die identisch oder voneinander verschieden sind, ein Wasserstoffatom, ein Halogenatom, eine geradkettige oder verzweigte Alkylgruppe mit 1 bis 20 Kohlenstoffatomen oder eine Gruppe -OR₅;
- R₅ ein Wasserstoffatom, eine Alkylgruppe mit 1 bis 12 Kohlenstoffatomen oder eine Gruppe -COR₁₀, wobei R₁₀ die nachfolgend angegebenen Bedeutungen aufweist;
- R₆:
(a) ein Wasserstoffatom,
(b) eine Alkylgruppe mit 1 bis 12 Kohlenstoffatomen,
(c) eine Gruppe der Formel: 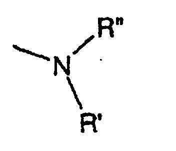 worin R' und R" die nachfolgend angegebenen Bedeutungen aufweisen,
(d) eine Gruppe -OR₁₁, worin R₁₁ die nachfolgend angegebene Bedeutung hat;
- R₈ und R₉, die identisch oder voneinander verschieden sind, ein Wasserstoffatom oder eine Alkylgruppe mit 1 bis 12 Kohlenstoffatomen;
- R₁₀ eine Alkylgruppe mit 1 bis 12 Kohlenstoffatomen;
- R₁₁ ein Wasserstoffatom, eine geradkettige oder verzweigte Alkylgruppe mit 1 bis 20 Kohlenstoffatomen, eine Alkenylgruppe, eine Mono- oder Polyhydroxyalkylgruppe, eine Arylgruppe oder eine Aralkylgruppe, die gegebenenfalls mit mindestens einer der folgenden Gruppen substituiert ist (sind): Halogen, Hydroxy oder Nitro, einen Zuckerrest, einen Aminosäurerest oder einen Peptidrest;
- R' und R", die identisch oder voneinander verschieden sind, Wasserstoff, eine Alkylgruppe mit 1 bis 12 Kohlenstoffatomen, eine Mono- oder Polyhydroxyalkylgruppe, eine Arylgruppe, die gegebenenfalls mit mindestens einem Halogenatom, mindestens einer Hydroxygruppe oder mindestens einer Nitrogruppe substituiert ist, oder einen Aminosäurerest oder einen Zuckerrest; oder R' und R" bilden gemeinsam einen Heterocyclus.
und die Salze und die optischen und geometrischen Isomere dieser Verbindungen.

2. Verbindungen nach Anspruch 1, **dadurch gekennzeichnet, daß** sie in Form von Alkalimetallsalzen, Erdalkalimetallsalzen, Zinksalzen oder Salzen von organischen Aminen vorliegen.

3. Verbindungen nach Anspruch 1, **dadurch gekennzeichnet, daß** sie unter den folgenden Verbindungen oder deren Gemischen ausgewählt sind:
- 5-(3-*t*-Butyl-4-methoxyphenyl)-2-thiophenacrylsäure,
- 5-(3-*t*-Butyl-4-methoxyphenyl)-2-thiophenpropiolsäure,
- 2-(3-*t*-Butyl-4-methoxyphenyl)-4-thiophenacrylsäure,
- 4-(3-*t*-Butyl-4-methoxyphenyl)-2-thiophenacrylsäure,
- 5-(3,5,5,8,8-Pentamethyl-5,6,7,8-tetrahydro-2-naphthyl)-2-thiophenacrylsäure,
- 4-(3,5,5,8,8-Pentamethyl-5,6,7,8-tetrahydro-2-naphthyl)-2-thiophenacrylsäure,
- 4-(5,6,7,8-Tetrahydro-5,5,8,8-tetramethyl-2-naphthyl)-2-thiophenacrylsäure,
- 5-(3,5,5,8,8-Pentamethyl-5,6,7,8-tetrahydro-2-naphthyl)-2-thiophenpropiolsäure,
- 3-(3,5,5,8,8-Pentamethyl-5,6,7,8-tetrahydro-2-naphthyl)phenylpropiolsäure,
- N-Methyl-4-(5,6,7,8-tetrahydro-5,5,8,8-tetramethyl-2-naphthyl)-2-pyrrolacrylsäure,
- 3-(5,6,7,8-Tetrahydro-5,5,8,8-tetramethyl-2-naphthyl)phenylacrylsäure,
- N-Methyl-4-(3,5,5,8,8-pentamethyl-5,6,7,8-tetrahydro-2-naphthyl)-2-pyrrolacrylsäure,
- 4-(3,5,5,8,8-Pentamethyl-5,6,7,8-tetrahydro-2-naphthyl)-2-pyrrolacrylsäure,
- 3-(3,5,5,8,8-Pentamethyl-5,6,7,8-tetrahydro-2-naphthyl)phenylacrylsäure,
- 3-(5,6,7,8-Tetrahydro-5,5,8,8-tetramethyl-2-naphthyl)phenylpropiolsäure,
- 2-Methoxy-3-(3,5,5,8,8-pentamethyl-5,6,7,8-tetrahydro-2-naphthyl)phenylacrylsäure,
- 2-Propyloxy-3-(3,5,5,8,8-pentamethyl-5,6,7,8-tetrahydro-2-naphthyl)phenylacrylsäure,
- 2-Heptyloxy-3-(3,5,5,8,8-pentametnyl-5,6,7,8-tetrahydro-2-naphthyl)phenylacrylsäure,
- 2-Methoxymethoxy-3-(3,5,5,8,8-pentamethyl-5,6,7,8-tetrahydro-2-naphthyl)phenylacrylsäure,
- 2-Hydroxy-3-(3,5,5,8,8-pentamethyl-5,6,7,8-tetrahydro-2-naphthyl)phenylacrylsäure,
- 3-(3-Methoxy-5,6,7,8-tetrahydro-5,5,8,8-tetramethyl-2-naphthyl)phenylacrylsäure,
- 3-(3-Propyloxy-5,6,7,8-tetrahydro-5,5,8,8-tetramethyl-2-naphthyl)phenylacrylsäure,
- 3-(3-Heptyloxy-5,6,7,8-tetrahydro-5,5,8,8-tetramethyl-2-naphthyl)phenyläcrylsäure,
- 3-(3-Methoxymethoxy-5,6,7,8-tetrahydro-5,5,8,8-tetramethyl-2-naphthyl)phenylacrylsäure,
- 3-(3-Hydroxy-5,6,7,8-tetrahydro-5,5,8,8-tetramethyl-2-naphthyl)phenylacrylsäure,
- 3-(4,4,7-Trimethylthiochroman-6-yl)phenylacrylsäure,
- N-Ethyl-3-(3,5,5,8,8-pentamethyl-5,6,7,8-tetrahydro-2-naphthyl)-phenylacrylamid,
- N-(4-Hydroxyphenyl)-3-(3,5,5,8,8-pentamethyl-5,6,7,8-tetrahydro-2-naphthyl)-phenylacrylamid,
- 3-(3,5,5,8,8-Pentamethyl-5,6,7,8-tetrahydro-2-naphthyl)phenylacrylsäuremorpholid,
- Ethyl-3-(3,5,5,8,8-pentamethyl-5,6,7,8-tetrahydro-2-naphthyl)phenylacrylat,
- 3-[3-(3,5,5,8,8-Pentamethyl-5,6,7,8-tetrahydro-2-naphthyl)phenyl]but-2-ensäure,
- 4-Methoxymethoxy-3-(3,5,5,8,8-pentamethyl-5,6,7,8-tetrahydro-2-naphthyl)phenylacrylsäure,
- 4-Hydroxy-3-(3,5,5,8,8-pentamethyl-5,6,7,8-tetrahydro-2-naphthyl)phenylacrylsäure,
- 4-Propyloxy-3-(3,5,5,8,8-pentamethyl-5,6,7,8-tetrahydro-2-naphthyl)phenylacrylsäure,
- 4-Heptyloxy-3-(3,5,5,8,8-pentamethyl-5,6,7,8-tetrahydro-2-naphthyl)phenylacrylsäure,
- 3-(3,5,5,8,8-Pentamethyl-5,6,7,8-tetrahydro-2-naphthyl)phenylacrolein,
- 3-[3-(3,5,5,8,8-Pentamethyl-5,6,7,8-tetrahydro-2-naphthyl)phenyl]prop-2-en-1-ol,
- *cis*-3-[3-(3,5,5,8,8-Pentamethyl-5,6,7,8-tetrahydro-2-naphthyl)phenyl]but-2-ensäure,
- *cis*-3-[3-(3,5,5,8,8-Pentamethyl-5,6,7,8-tetrahydro-2-naphthyl)phenyl]acrylsäure,
- 5-(3,5,5,8,8-Pentamethyl-5,6,7,8-tetrahydro-2-naphthyl)-3-pyridinacrylsäure,
- 3-(3-Butyl-5,6,7,8-tetrahydro-5,5,8,8-tetramethyl-2-naphthyl)phenylacrylsäure, und
- 6-(3,5,5,8,8-Pentamethyl-5,6,7,8-tetrahydro-2-naphthyl)-2-pyridinacrylsäure.

4. Verbindungen nach Anspruch 1, **dadurch gekennzeichnet, daß** sie mindestens eine und vorzugsweise alle folgenden Eigenschaften aufweisen:
- R₁ ist eine Gruppe -CO-R₆,
- Ar₂ bedeutet die Gruppe der Formel (a) oder (d),
- X ist die folgende Gruppe: 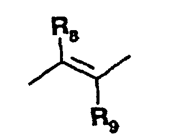
- die Gruppen R₂ und R₃ bilden gemeinsam mit dem angrenzenden aromatischen Ring einen 5- oder 6-gliedrigen Ring, der gegebenenfalls mit Methylgruppen substituiert und/oder gegebenenfalls durch ein Sauerstoff- oder Schwefelatom unterbrochen ist.

5. Verbindungen nach Anspruch 1, **dadurch gekennzeichnet, daß** es sich bei der Verbindung um die 4-Hydroxy-3-(3,5,5,8,8-pentamethyl-5,6,7,8-tetrahydro-2-naphthyl)phenylacrylsäure handelt.

6. Verbindung nach Anspruch 5 als Arzneimittel.

7. Verwendung der Verbindung des Anspruchs 6 zur Herstellung eines Arzneimittels, das zur Behandlung von dermatologischen Erkrankungen, die mit einer Verhornungsstörung einhergehen, welche mit der Differenzierung und Proliferation zusammenhängt, insbesondere zur Behandlung von Acne vulgaris, Acne comedonica, polymorpher Acne, Acne rosaceae, nodulocystischer Acne, Acne conglobata, Acne senilis, sekundären Akneformen, wie Acne solaris, Acne medikamentosa oder Acne professionalis; zur Behandlung von weiteren Arten von Keratinisierungsstörungen, insbesondere Ichtyosis, ichtyosisartigen Zuständen, Darier Krankheit, Palmoplantarkeratosen, Leukoplakien und leukoplakieformen Zuständen und Lichen der Haut oder der Schleimhäute (buccalis); zur Behandlung von weiteren dermatologischen Erkrankungen, die mit einer Keratinisierungsstörung mit entzündlicher und/oder immuno allergischer Komponente verbunden sind, und insbesondere allen Arten von Psoriasis der Haut, der Schleimhäute oder der Nägel, Psoriasis arthropathica, Atopie der Haut, beispielsweise Ekzemen, oder Atopie der Atemwege oder auch Hypertrophie des Zahnfleisches; verschiedenen entzündlichen Erkrankungen, die mit keinen Keratinisierungsstörungen verbunden sind; zur Behandlung von allen Proliferationen der Dermis oder Epidermis, die gutartig oder bösartig und gegebenenfalls viralen Ursprungs sein können, beispielsweise Verrucae vulgares, Verrucae planae und Epidermodysplasia verruciformis, Papillomatosis oralis oder florida und Proliferationen, die durch UV-Strahlung hervorgerufen werden können, insbesondere im Falle von Epithelioma basocellulare und spinocellulare; zur Behandlung von weiteren dermatologischen Erkrankungen, wie beispielsweise bullösen Dermatosen und Erkrankungen des Kollagens; zur Behandlung von verschiedenen ophthalmologischen Störungen, insbesondere Corneopathien; zur Vermeidung und Bekämpfung der Hautalterung, die lichtinduziert oder altersbedingt sein kann, oder zur Verminderung von Pigmentierungen und aktinischen Keratosen oder allen Erkrankungen, die mit der altersbedingten oder aktinischen Hautalterung verbunden sind; zur Vorbeugung oder zur Bekämpfung von Wundmalen der epidermalen und/oder dermalen Atrophie, die durch lokal oder systemisch verabreichte Corticosteroide hervorgerufen wird, oder beliebigen weiteren Formen der Atrophie der Haut, zur Vorbeugung oder zur Behandlung von Störungen der Wundheilung oder zur Vorbeugung oder Behandlung von Streifen; zur Förderung der Narbenbildung; zur Bekämpfung von Funktionsstörungen der Talgdrüsen, beispielsweise Hyperseborrhoe bei Akne oder Seborrhoe simplex; zur Behandlung oder Vorbeugung von krebsartigen oder präcancerösen Zuständen und insbesondere promyelocytärer Leukämie; zur Behandlung von entzündlichen Erkrankungen, wie Arthritis, zur Behandlung von beliebigen Erkrankungen viralen Ursprungs der Haut oder allgemeinen viralen Erkrankungen; zur Vorbeugung oder Behandlung der Alopezie; zur Behandlung von dermatologischen Erkrankungen mit Immunkomponente; zur Behandlung von Erkrankungen des cardiovasculären Systems, beispielsweise Arteriosklerose oder Bluthochdruck sowie nicht insulinpflichtiger Diabetes und zur Behandlung von Hautstörungen, die von einer Exposition gegenüber UV-Strahlung herrühren, vorgesehen ist.

8. Verbindungen nach einem der Ansprüche 1 bis 4 als Arzneimittel.

9. Verwendung einer Verbindung nach Anspruch 8 zur Herstellung eines Arzneimittels, das zur Behandlung von dermatologischen Erkrankungen, die mit einer Verhomungsstörung einhergehen, welche mit der Differenzierung und Proliferation zusammenhängt, insbesondere zur Behandlung von Acne vulgaris, Acne comedonica, polymorpher Acne, Acne rosaceae, nodulocystischer Acne, Acne conglobata, Acne senilis, sekundären Akneformen, wie Acne solaris, Acne medikamentosa oder Acne professionalis; zur Behandlung von weiteren Arten von Keratinisierungsstörungen, insbesondere Ichtyosis, ichtyosisartigen Zuständen, Darier Krankheit, Palmoplantarkeratosen, Leukoplakien und leukoplakieformen Zuständen und Lichen der Haut oder der Schleimhäute (buccalis); zur Behandlung von weiteren dermatologischen Erkrankungen, die mit einer Keratinisierungsstörung mit entzündlicher und/oder immunoallergischer Komponente verbunden sind, und insbesondere allen Arten von Psoriasis der Haut, der Schleimhäute oder der Nägel, Psoriasis arthropathica, Atopie der Haut, beispielsweise Ekzemen, oder Atopie der Atemwege oder auch Hypertrophie des Zahnfleisches; verschiedenen entzündlichen Erkrankungen, die mit keinen Keratinisierungsstörungen verbunden sind; zur Behandlung von allen Proliferationen der Dermis oder Epidermis, die gutartig oder bösartig und gegebenenfalls viralen Ursprungs sein können, beispielsweise Verrucae vulgares, Verrucae planae und Epidermodysplasia verruciformis, Papillomatosis oralis oder florida und Proliferationen, die durch UV-Strahlung hervorgerufen werden können, insbesondere im Falle von Epithelioma basocellulare und spinocellulare; zur Behandlung von weiteren dermatologischen Erkrankungen, wie beispielsweise bullösen Dermatosen und Erkrankungen des Kollagens; zur Behandlung von verschiedenen ophthalmologischen Störungen, insbesondere Corneopathien; zur Vermeidung und Bekämpfung der Hautalterung, die lichtinduziert oder altersbedingt sein kann, oder zur Verminderung von Pigmentierungen und aktinischen Keratosen oder allen Erkrankungen, die mit der altersbedingten oder aktinischen Hautalterung verbunden sind; zur Vorbeugung oder zur Bekämpfung von Wundmalen der epidermalen und/oder dermalen Atrophie, die durch lokal oder systemisch verabreichte Corticosteroide hervorgerufen wird, oder beliebigen weiteren Formen der Atrophie der Haut, zur Vorbeugung oder Behandlung von Störungen der Wundheilung oder zur Vorbeugung oder Behandlung von Streifen; zur Förderung der Narbenbildung; zur Bekämpfung von Funktionsstörungen der Talgdrüsen, beispielsweise Hyperseborrhoe bei Akne oder Seborrhoe simplex; zur Behandlung oder Vorbeugung von krebsartigen oder präcancerösen Zuständen und insbesondere promyelocytärer Leukämie; zur Behandlung von entzündlichen Erkrankungen, wie Arthritis, zur Behandlung von beliebigen Erkrankungen viralen Ursprungs der Haut oder allgemeinen viralen Erkrankungen; zur Vorbeugung oder Behandlung der Alopezie; zur Behandlung von dermatologischen Erkrankungen mit Immunkomponente; zur Behandlung von Erkrankungen des cardiovasculären Systems, beispielsweise Arteriosklerose oder Bluthochdruck sowie nicht insulinpflichtiger Diabetes und zur Behandlung von Hautstörungen, die von einer Exposition gegenüber UV-Strahlung herrühren, vorgesehen ist.

10. Pharmazeutische Zusammensetzung, **dadurch gekennzeichnet, daß** sie in einem pharmazeutisch akzeptablen Träger mindestens eine Verbindung nach einem der Ansprüche 1 bis 5 enthält.

11. Zusammensetzung nach Anspruch 10, **dadurch gekennzeichnet, daß** die Konzentration der Verbindung(en) nach einem der Ansprüche 1 bis 5 im Bereich von 0,001 bis 5 Gew.-%, bezogen auf die gesamte Zusammensetzung, liegt.

12. Kosmetische Zusammensetzung, **dadurch gekennzeichnet, daß** sie in einem kosmetisch akzeptablen Träger mindestens eine Verbindung nach einem der Ansprüche 1 bis 5 enthält.

13. Zusammensetzung nach Anspruch 12, **dadurch gekennzeichnet, daß** die Konzentration der Verbindung(en) nach einem der Ansprüche 1 bis 5 im Bereich von 0,001 bis 3 Gew.-%, bezogen auf die gesamte Zusammensetzung, liegt.

14. Verwendung einer Zusammensetzung nach einem der Ansprüche 12 oder 13 zur Herstellung einer Zusammensetzung für die Körper- oder Haarpflege.
